# EUROPEAN PATENT SPECIFICATION

(11) **EP 4 431 025 B1**
(45) Date of publication and mention of the grant of the patent: **11.06.2025**
(21) Application number: 24163819.6
(22) Date of filing: 15.03.2024
(51) Int. Cl.: A61B 8/08

(54) **PROCESSING APPARATUS AND COMPUTER PROGRAM**
VERARBEITUNGSVORRICHTUNG UND COMPUTERPROGRAMM
APPAREIL DE TRAITEMENT ET PROGRAMME INFORMATIQUE

(30) Priority: 16.03.2023 US 202363452536 P
(43) Date of publication of application: 18.09.2024
(73) Proprietor: Olympus Corporation, Hachioji-shi Tokyo 192-8507 (JP)
(72) Inventor: INAGAKI, Genri, Tokyo, 192-8507 (JP); TAKAZAWA, Naohiro, Tokyo, 192-8507 (JP); OGAWA, Ryohei, Tokyo, 192-8507 (JP); MIDORIKAWA, Masamichi, Tokyo, 192-8507 (JP); NISHIMURA, Hidetoshi, Tokyo, 192-8507 (JP); MILFORD, Jordan, Center Valley, PA, 18034 (US); HORIO, Hirokazu, Center Valley, PA, 18034 (US); MINO, Hiroyuki, Center Valley, PA, 18034-0610 (US)
(74) Representative: Schicker, Silvia

(56) References cited:
- US-A1- 2003 078 502
- US-A1- 2020 078 103
- US-A1- 2021 259 660

## Description

### BACKGROUND

In a manipulation using an endoscope, known is an endoscope system that generates diagnosis support information based on an image captured by the endoscope to support a user. US 2019/0247127 A1 discloses a method of recognizing a tissue or the like displayed in an ultrasonic image captured by an ultrasonic endoscope and displaying information regarding the recognized tissue as being superimposed on the ultrasonic image. US 2020/078103 A1 discloses a window displaying a target location, indicators, which indicate stored previous needle trajectories and/or a current needle trajectory based on the current position and orientation of an elongated device, from which the respective needle extends, and a range of motion limit indicator of the elongated device, wherein the range of motion limit indicator indicates that the elongated device cannot or should not be steered any further in a particular direction. US 2021/259660 A1 discloses angles of the needle to the patient's skin at a needle entry point as well as deep-learning-based techniques to determine locations in image data.

Document US 2003/078502 A1 discloses a processing apparatus configured to acquire an image in which region marker information is set with respect to an ultrasonic endoscope with a biopsy needle, the needle being movable within a given range and the processing apparatus being configured to calculate the angle the needle for inserting the needle into the lesion based on the needle range and the marker information.

### SUMMARY

In accordance with the one aspect of the invention, there is provided a processing apparatus as defined in claim 1.

In accordance with the other aspect of the invention, there is provided a computer program as defined in claim 9.

### BRIEF DESCRIPTION OF THE DRAWINGS

FIG. 1 is a diagram for describing a configuration example of an endoscope system.
FIG. 2 is a diagram for describing another configuration example of the endoscope system.
FIG. 3 is a diagram for describing another configuration example of the endoscope system.
FIG. 4 is a diagram for describing an example of a distal end portion or the like.
FIG. 5 is a diagram for describing an example of a probe and a raising base of a treatment tool.
FIG. 6 is a diagram for describing an example of an ultrasound image and a movable range image.
FIG. 7 is a flowchart describing an example of a manipulation according to the present embodiment.
FIG. 8 is a diagram for describing an example of a method of calculating an angle of a biopsy needle.
FIG. 9 is a diagram for describing another example of the movable range image.
FIG. 10 is a diagram for describing another configuration example of the endoscope system.
FIG. 11 is a diagram for describing an example of a neural network.
FIG. 12 is a diagram for describing another example of the neural network and an example of region marker information.
FIG. 13 is a diagram for describing another example of the region marker information.
FIG. 14 is a diagram for describing another example of the region marker information.
FIG. 15 is a diagram for describing a configuration example of a training device.
FIG. 16 is a diagram for describing a pressing pressure.
FIG. 17 is a diagram for describing another configuration example of the endoscope system.
FIG. 18 is a flowchart describing an example of processing performed in a biopsy.
FIG. 19 is a flowchart describing a processing example of determination processing.
FIG. 20 is a diagram for describing a method of determining whether or not a pressure is an appropriate pressure.
FIG. 21 is a diagram for describing a configuration example of a motorized endoscope system.
FIG. 22 is a diagram for describing a configuration example of a drive control device.
FIG. 23 is a diagram for describing a curved portion and a drive mechanism for the curved portion.
FIG. 24 is a diagram for describing a configuration example of an advance/retreat drive device.
FIG. 25 is a diagram for describing a configuration example of a coupling element including a roll drive device.
FIG. 26 is a diagram for describing a configuration example of an inference section.
FIG. 27 is a diagram for describing another configuration example of the inference section.
FIG. 28 is a diagram for describing an example of position information and direction information of a distal end portion.
FIG. 29 is a diagram for describing another configuration example of the inference section.
FIG. 30 is a diagram for describing another configuration example of the inference section.
FIG. 31 is a flowchart describing a processing example of presentation processing for presenting operation support information.
FIG. 32 is a flowchart describing a processing example of first notification.
FIG. 33 is a diagram for describing a notification example of the first notification.
FIG. 34 is a flowchart describing a processing example of second notification.
FIG. 35 is a diagram for describing a notification example of the second notification.
FIG. 36 is a flowchart describing a processing example of third notification.
FIG. 37 is a diagram for describing a notification example of the third notification.
FIG. 38 is a diagram for describing a modification of operation support information.

### DETAILED DESCRIPTION

The following disclosure provides many different embodiments, or examples, for implementing different features of the provided subject matter. These are, of course, merely examples and are not intended to be limiting. In addition, the disclosure may repeat reference numerals and/or letters in the various examples. This repetition is for the purpose of simplicity and clarity and does not in itself dictate a relationship between the various embodiments and/or configurations discussed. Further, when a first element is described as being "connected" or "coupled" to a second element, such description includes embodiments in which the first and second elements are directly connected or coupled to each other, and also includes embodiments in which the first and second elements are indirectly connected or coupled to each other with one or more other intervening elements in between.

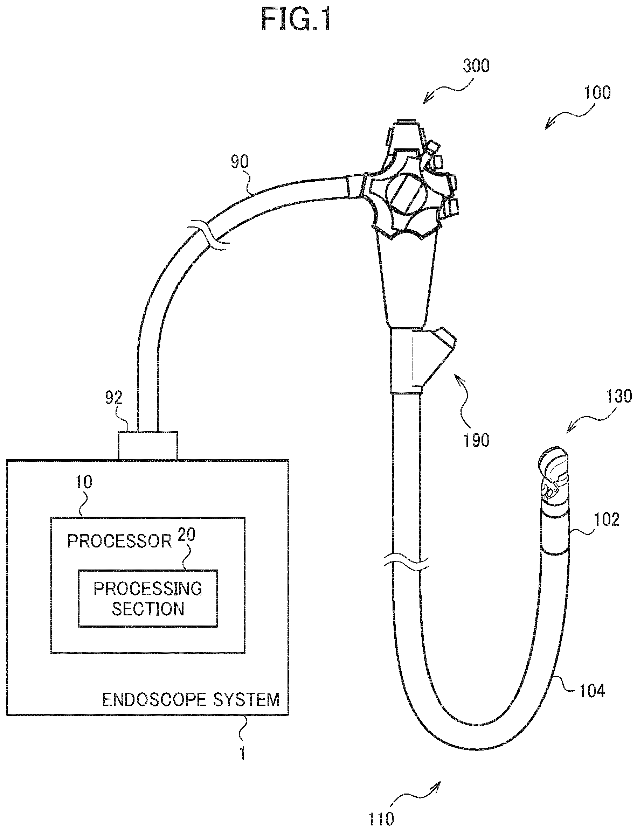

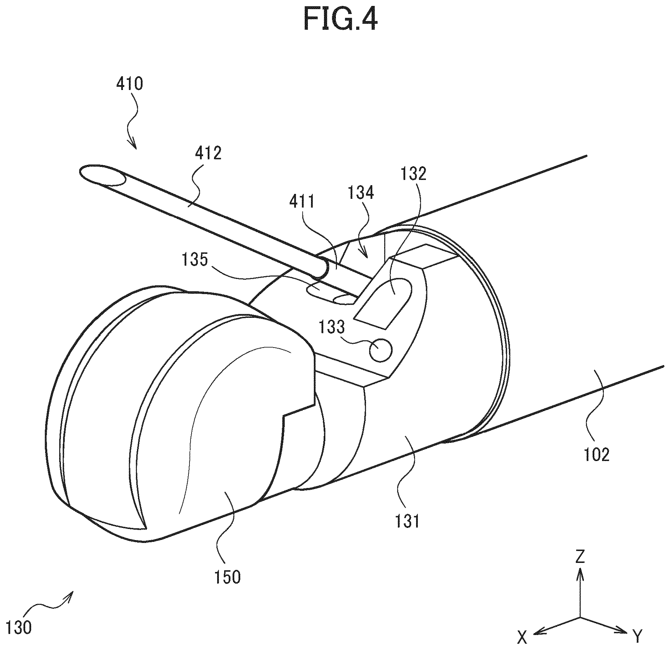

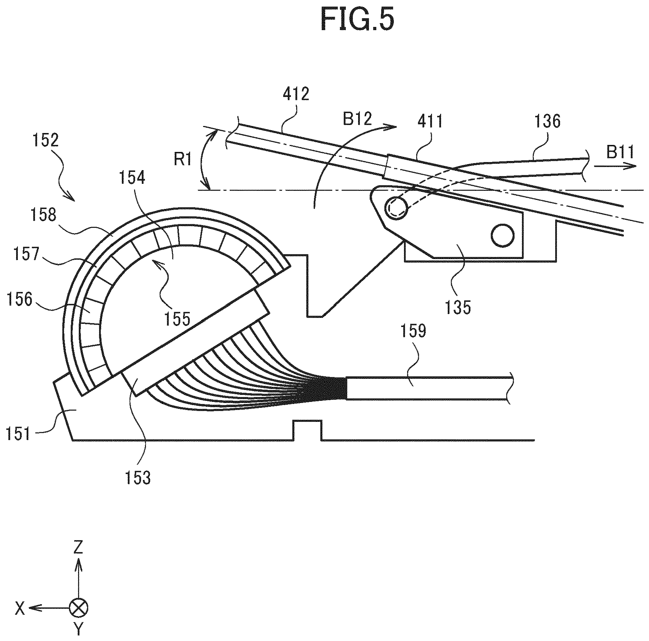

A configuration example of an endoscope system 1 according to the present embodiment is described with reference to FIG. 1. As described later in detail, the endoscope system 1 that makes an ultrasonic diagnosis of the inside of the body of a subject using an ultrasonic endoscope 100 and that functions as a medical ultrasonic endoscope system is given as an example in the present embodiment, but the whole or part of a method according to the present embodiment may be applied to, for example, an endoscope without an ultrasonic diagnosis function, an industrial endoscope, or the like. The subject is, for example, a patient, but a main subject that is subjected to an ultrasonic diagnosis is collectively expressed as the subject in the present embodiment. In the following description, a convergent beam of ultrasonic waves used in the ultrasonic diagnosis is simply referred to as ultrasonic waves or a beam. A type of the ultrasonic endoscope 100 to which the method according to the present embodiment is applied is exemplified by a convex-type ultrasonic endoscope based on a scan method for performing scan along a convex surface with a beam, but the method according to the present embodiment is not prevented from being applied to the ultrasonic endoscope 100 of a sector-type, a linear-type, a radial-type, or the like. Note that the ultrasonic endoscope 100 of the convex-type will be described later with reference to FIGS. 4 and 5.

The endoscope system 1 according to the present embodiment includes a processor 10. The processor 10 according to the present embodiment has the following hardware configuration. The hardware can include at least one of a circuit that processes a digital signal or a circuit that processes an analog signal. For example, the hardware can include one or more circuit devices mounted on a circuit board, or one or more circuit elements. The one or more circuit devices are, for example, integrated circuits (ICs) or the like. The one or more circuit elements are, for example, resistors, capacitors, or the like.

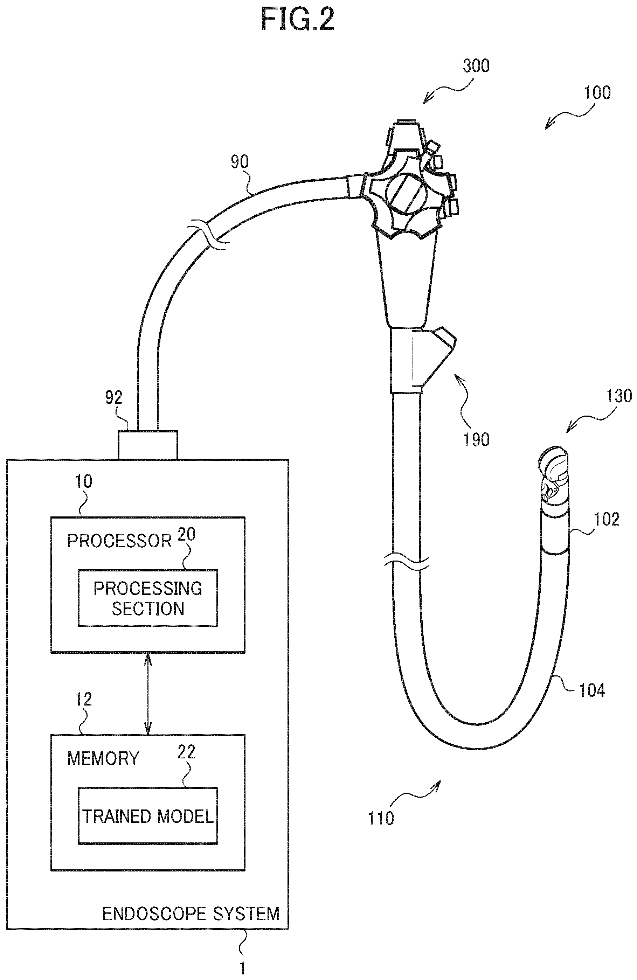

For example, the endoscope system 1 according to the present embodiment may include a memory 12, which is not illustrated in FIG. 1, and the processor 10 that operates based on information stored in the memory 12. With this configuration, the processor 10 can function as a processing section 20. The information is, for example, a program, various kinds of data, and the like. The program may include, for example, a trained model 22, which will be described later with reference to FIG. 2. A central processing unit (CPU), a graphics processing unit (GPU), a digital signal processor (DSP), or the like can be used as the processor 10. The memory 12 may be a semiconductor memory such as a static random access memory (SRAM) and a dynamic random access memory (DRAM). The memory 12 may be a register. The memory 12 may be a magnetic storage device such as a hard disk device. The memory 12 may be an optical storage device such as an optical disk device. For example, the memory 12 stores a computer-readable instruction. The instruction is executed by the processor 10, whereby functions of sections of the processing section 20 are implemented as processing. The instruction mentioned herein may be an instruction set that is included in the program, or may be an instruction that instructs the hardware circuit included in the processor 10 to operate. The memory 12 is also referred to as a storage device. The main section that performs processing or the like according to the present embodiment is collectively referred to as the endoscope system 1 for descriptive convenience unless otherwise described, but can be replaced by hardware of the processor 10 as appropriate, and can also be replaced by software of the processing section 20 or each section included in the processing section 20 as appropriate.

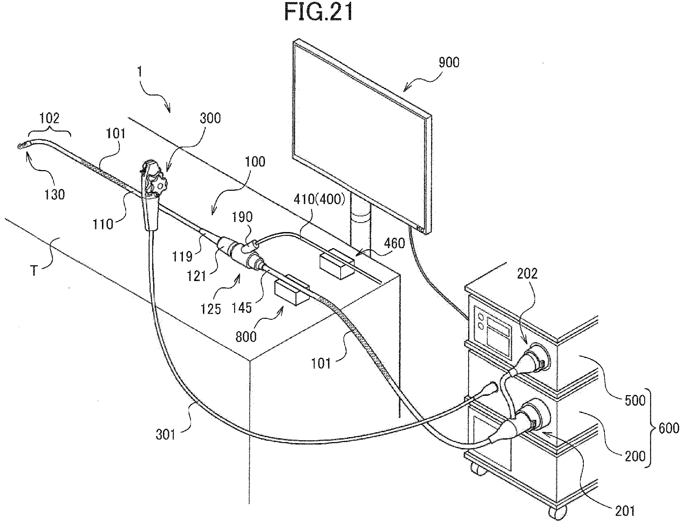

The ultrasonic endoscope 100 includes an insertion portion 110, an operation device 300, a universal cable 90 that extends from a side portion of the operation device 300, and a connector portion 92. In the following description, an insertion side of the insertion portion 110 into a lumen of a subject is referred to as a "distal end side", and a mounting side of the insertion portion 110 to the operation device 300 is referred to as a "base end side". In a case of the motorized endoscope system 1, which will be described later with reference to FIG. 21 or the like, the mounting side of the insertion portion 110 to a control device 600, which will be described later, is referred to as the "base end side". In a case of a biopsy needle 410, which will be described later, one side from an insertion opening 190 toward a distal end opening portion 134, which will be described later with reference to FIG. 4, is referred to as the "distal end side", and the other side from the distal end opening portion 134 toward the insertion opening 190 is referred to as the "base end side". In addition, the movement of the insertion portion 110 to the distal end side may be referred to as "advance", the movement of the insertion portion 110 to the base end side may be referred to as "retreat", and advance and retreat may be simply referred to as "advance/retreat". The same applies to the biopsy needle 410.

The insertion portion 110 is a portion that is inserted into the inside of the body of the subject. The insertion portion 110 is arranged on the distal end side, and includes a distal end portion 130, a curved portion 102, and a flexible portion 104. The distal end portion 130 holds an ultrasonic transducer unit 152, which will be described later, and has rigidity. The curved portion 102 is coupled to the base end side of the distal end portion 130 and can be curved. The flexible portion 104 is coupled to the base end side of the curved portion 102 and has flexibility. Note that the curved portion 102 may be electrically curved, which will be described in detail later with reference to FIG. 21 or the like. A plurality of signal lines that transmits electric signals or the like, an optical fiber cable bundle for illumination light, an air supply/aspiration tube, an ultrasonic cable 159, or the like is routed inside the insertion portion 110, and a treatment tool insertion path and the like are formed inside the insertion portion 110. The ultrasonic cable 159 will be described later with reference to FIG. 5. Note that the inside of the insertion portion 110 mentioned herein corresponds to an internal path 101, which will be described later with reference to FIG. 21.

The operation device 300 includes, in addition to an insertion opening 190, which will be described later, a plurality of operation members. The operation members are, for example, a raising base operation section that pulls a raising base operation wire 136, which will be described later, a pair of angle knobs that controls a curving angle of the curved portion 102, an air supply/water supply button, an aspiration button, and the like.

Through the universal cable 90, the plurality of signal lines that transmits electric signals or the like to the inside of the universal cable 90, the optical fiber cable bundle for illumination light, the air supply/aspiration tube, the ultrasonic cable 159, and the like are inserted. The ultrasonic cable 159 will be described later with reference to FIG. 5. A connector portion 92 is arranged at an end portion of the universal cable 90. The connector portion 92 is connected to an endoscope observation device, an ultrasonic observation device, a light source device, an air supply/water supply device, and the like, which are not illustrated. That is, the endoscope system 1 illustrated in FIG. 1 includes the endoscope observation device, the ultrasonic observation device, the light source device, the air supply/water supply device, and the like, which are not illustrated. For example, at least one of these devices may exist outside the endoscope system 1, and a plurality of connector portions 92 may exist.

The ultrasonic endoscope 100 converts electric pulse-type signals received from the ultrasonic observation device, which is not illustrated, into pulse-type ultrasonic waves using a probe 150 arranged in the distal end portion 130, which will be described later, irradiates the subject with the ultrasonic waves, converts the ultrasonic waves reflected by the subject into echo signals, which are electric signals expressed by a voltage change, and outputs the echo signals. For example, the ultrasonic endoscope 100 transmits the ultrasonic waves to tissues around a digestive tract or a respiratory organ, and receives the ultrasonic waves reflected on the tissues. The digestive tract is, for example, the esophagus, the stomach, the duodenum, the large intestine, or the like. The respiratory organ is, for example, the trachea, the bronchus, or the like. The issue is, for example, the pancreas, the gallbladder, the bile duct, the bile duct tract, lymph nodes, a mediastinum organ, blood vessels, or the like. The ultrasonic observation device, which is not illustrated, performs predetermined processing on the echo signals received from the probe 150 to generate ultrasonic image data. The predetermined processing mentioned herein is, for example, bandpass filtering, envelope demodulation, logarithm transformation, or the like.

The ultrasonic endoscope 100 of the present embodiment may further include an imaging optical system, which will be described later with reference to FIG. 4. With this configuration, the ultrasonic endoscope 100 is inserted into the digestive tract or respiratory organ of the subject, and is capable of capturing an image of the digestive tract, the respiratory organ, or the like.

The endoscope system 1 of the present embodiment may have a configuration in a configuration example illustrated in FIG. 2. That is, the endoscope system 1 of the present embodiment may further include the memory 12 that stores the trained model 22. Although details will be described later, the trained model 22 of the present embodiment is trained so as to output, to an ultrasonic image captured by the ultrasonic endoscope 100, region marker information of a detection target in the ultrasonic image.

Training in the present embodiment is, for example, machine learning as supervised learning. In the supervised learning, the trained model 22 is generated by supervised learning based on a dataset that associates input data and a correct label with each other. That is, the trained model 22 of the present embodiment is generated by, for example, supervised learning based on a dataset that associates input data including an ultrasonic image and a correct label including region marker information with each other. Examples of the dataset are not limited thereto, and details of the dataset will be described later.

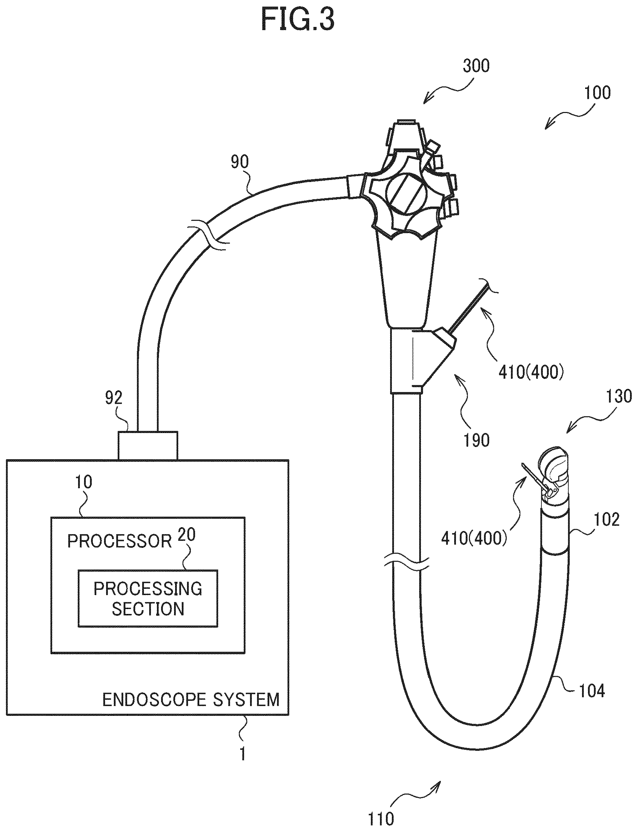

The endoscope system 1 of the present embodiment may have a configuration in a configuration example illustrated in FIG. 3. That is, the ultrasonic endoscope 100 connected to the endoscope system 1 of the present embodiment may include the biopsy needle 410 as a treatment tool 400. Note that the biopsy needle 410 will be given as an example in the following description, but another treatment tool 400 is not prevented from being applied to the endoscope system 1 of the present embodiment. As described above, the treatment tool insertion path is arranged inside the insertion portion 110, and the insertion opening 190 is connected to the treatment tool insertion path. With this configuration, the biopsy needle 410 inserted from the insertion opening 190 is led out from the distal end portion 130 via the treatment tool insertion path and the distal end opening portion 134, which will be described later. Note that details of the biopsy needle 410 will be described later with reference to FIG. 4. The endoscope system 1 of the present embodiment may have a configuration that combines the configuration example illustrated in FIG. 2 and the configuration example illustrated in FIG. 3.

A configuration example of the distal end portion 130 of the ultrasonic endoscope 100 will be described with reference to FIGS. 4 to 6. Note that the configuration of each portion of the distal end portion 130 according to the present embodiment is not limited to the following description, and can be modified in various manners. As a matter of descriptive convenience, X, Y, and Z axes are illustrated as three axes that are orthogonal to each other in FIG. 4 or subsequent drawings as appropriate. A direction along the X axis is referred to as an X axis direction, and is a direction along a longitudinal direction of the distal end portion 130. Assume that the distal end side is a +X direction, and the base end side is a -X direction. A direction along the Y axis is referred to as a Y axis direction, and a direction along the Z axis is referred to as a Z axis direction. In a case where an ultrasonic diagnosis is performed using an ultrasonic transducer array 155 in a one-dimensional array, which is exemplified in FIG. 5, each of the X axis direction, the Y axis direction, and the Z axis direction in FIGS. 4 and 5 can also be referred to as a scanning direction, a slice direction, and a distance direction. Assume that being "orthogonal" includes, in addition to being orthogonal at 90°, a case of being orthogonal at an angle somewhat inclined from 90°.

As illustrated in a perspective view in FIG. 4, the distal end portion 130 includes a main portion 131 and the probe 150 that projects toward the distal end side of the main portion 131. The main portion 131 includes an objective lens 132, an illumination lens 133, and the distal end opening portion 134. The objective lens 132 constitutes part of the imaging optical system, and captures light from the outside. The imaging optical system mentioned herein is an imaging sensor including a charge-coupled device (CCD), a complementary metal-oxide semiconductor (CMOS) sensor, or the like, an imaging module including an optical member, or the like, and can also be referred to as an imager, an image sensor, or a camera module. In the present embodiment, an endoscope image captured by the imaging optical system can also be referred to as an in-vivo image. The illumination lens 133 condenses illumination light and emits the illumination light to the outside. The distal end opening portion 134 is an outlet of the treatment tool insertion path. Although not illustrated in FIG. 4, the distal end portion 130 may additionally include an air supply/water supply nozzle or the like.

The probe 150 and a raising base 135 are now described with reference to FIG. 5. In FIG. 5, a configuration is added to a cross-sectional view at the center of the distal end portion 130 in the Y axis direction as appropriate, and a configuration that is unnecessary for description is deleted as appropriate for descriptive convenience. As illustrated in FIG. 5, the raising base 135 is arranged in the distal end opening portion 134 of the main portion 131. FIG. 5 illustrates that, at a basic position of the raising base 135, the longitudinal direction of the raising base 135 is not matched with the longitudinal direction of the distal end portion 130, and is inclined by an angle indicated by R1 from a longitudinal axis of the distal end portion 130. However, for example, at the basic position of the raising base 135, the longitudinal direction of the raising base 135 and the longitudinal direction of the distal end portion 130 may be parallel. Being parallel mentioned herein includes being substantially parallel, and the same applies to the following description. The longitudinal axis of the distal end portion 130 is an axis along the longitudinal direction of the distal end portion 130, and is an axis that is parallel to the X axis illustrated in FIG. 4 or the like. In the present embodiment, assume that a projection direction of the biopsy needle 410 can be regarded as being parallel to the longitudinal direction of the raising base 135. That is, in FIG. 5, the direction in which the biopsy needle 410 projects based on the basic position of the raising base 135 is inclined by the angle indicated by R1 from the longitudinal axis of the distal end portion 130. In the following description, an angle formed between the longitudinal direction of the raising base 135 and the longitudinal direction of the distal end portion 130 is hereinafter referred to as an inclination angle of the raising base 135. In addition, an angle in the projection direction of the biopsy needle 410 based on the longitudinal direction of the distal end portion 130 is simply referred to as an angle of the biopsy needle 410. That is, since the angle of the biopsy needle 410 is identical to the inclination angle of the raising base 135, the endoscope system 1 performs measurement or the like of the inclination angle of the raising base 135, and can thereby grasp the angle of the biopsy needle 410.

The raising base operation wire 136 is connected to the raising base 135. The user operates the raising base operation section, which is not illustrated, whereby the raising base operation wire 136 is pulled in a direction indicated by B11. As a result, the inclination angle of the raising base 135 changes in a direction indicated by B12. This allows the user to adjust a led-out angle of the biopsy needle 410. The raising base operation section, which is not illustrated, is included in, for example, the operation device 300 or the like. In the following description, an operator as a main person who operates the operation device 300 or the like is collectively expressed as the user. In the case of FIG. 5, the user operates the raising base operation section, which is not illustrated, whereby the inclination angle of the raising base 135 changes to be an angle larger than the angle indicated by R1.

The endoscope system 1 of the present embodiment may be capable of grasping the inclination angle of the raising base 135. For example, the endoscope system 1 measures the inclination angle of the raising base 135 with an angle sensor, which is not illustrated, and can thereby grasp the inclination angle of the raising base 135. Alternatively, the endoscope system 1 measures an operation amount of the raising base operation wire 136 using a position sensor, which is not illustrated, and uses a first table that associates the operation amount of the raising base operation wire 136 and the inclination angle of the raising base 135 with each other to grasp the inclination angle of the raising base 135. The raising base operation section, which is not illustrated, may be configured to control a stepping motor that pulls the raising base operation wire 136, and a table that associates the number of steps of the stepping motor and the inclination angle of the raising base 135 may serve as the first table. With this configuration, the endoscope system 1 is capable of grasping the inclination angle of the raising base 135, that is, the angle of the biopsy needle 410 in association with control of the raising base operation wire 136.

The probe 150 includes a housing 151 and the ultrasonic transducer unit 152, as illustrated in FIG. 5. The ultrasonic transducer unit 152 is engaged with the housing 151 and fixed. The ultrasonic transducer unit 152 includes a wiring substrate 153, a backing material 154, the ultrasonic transducer array 155, an acoustic matching layer 157, and an acoustic lens 158. The ultrasonic transducer array 155 includes a plurality of ultrasonic transducers 156. The ultrasonic transducer unit 152 having the above-mentioned configuration functions as the probe 150. Although not illustrated, in a case where an internal space exists in the housing 151, the ultrasonic transducer unit 152 may further include a filling portion that fills the internal space. For the filling portion, a material that is identical to the backing material 154 may be used, or another member having a heat dissipation property may be used.

The wiring substrate 153 functions as a relay substrate that relays the ultrasonic observation device, which is not illustrated, and the ultrasonic transducer array 155. That is, the wiring substrate 153 is electrically connected to each wire included in the ultrasonic cable 159 via an electrode, which is not illustrated, and is electrically connected to the corresponding ultrasonic transducer 156 via an electrode, which is not illustrated, a signal line, or the like. The wiring substrate 153 may be a rigid substrate or a flexible substrate.

The backing material 154 mechanically supports the ultrasonic transducer array 155, and also attenuates ultrasonic waves that propagate from the ultrasonic transducer array 155 to the inside of the probe 150. The backing material 154 is formed of, for example, a material having rigidity such as hard rubber, or the material may further contain, for example, ferrite, ceramic, or the like to form the backing material 154. This configuration can more effectively attenuate ultrasonic waves that propagate to the inside of the probe 150.

The ultrasonic transducer array 155 is configured so that the plurality of ultrasonic transducers 156 is arrayed at regular intervals in a one-dimensional array to form a convex curve shape along the X axis direction. The ultrasonic transducers 156 that constitute the ultrasonic transducer array 155 can be implemented by, for example, a piezoelectric element formed of piezoelectric ceramic represented by lead zirconate titanate (PZT), a piezoelectric polymer material represented by polyvinylidene difluoride (PVDF), or the like. In each ultrasonic transducer 156, a first electrode and a second electrode, which are not illustrated, are formed. The first electrode is electrically connected to the corresponding wire of the ultrasonic cable 159 via a signal line or the like on the wiring substrate 153. The signal line is not illustrated. The second electrode is connected to a ground electrode on the wiring substrate 153. The ground electrode is not illustrated. With this configuration, the ultrasonic transducers 156 can be sequentially driven based on a drive signal input by an electronic switch such as a multiplexer. With this configuration, the piezoelectric elements that constitute the ultrasonic transducers 156 are oscillated, whereby ultrasonic waves can be sequentially generated. In the ultrasonic transducer array 155, for example, the plurality of ultrasonic transducers 156 may be arrayed in a two-dimensional array or the like, and ultrasonic transducer array 155 can be modified in various manners.

The acoustic matching layer 157 is laminated outside the ultrasonic transducer array 155. A value of acoustic impedance of the acoustic matching layer 157 is within a range between a value of acoustic impedance of the ultrasonic transducer 156 and a value of acoustic impedance of the subject. This configuration allows ultrasonic waves to effectively penetrate the subject. The acoustic matching layer 157 is formed of, for example, an organic material such as an epoxy resin, a silicon rubber, polyimide, and polyethylene. Note that the acoustic matching layer 157 is illustrated as one layer for convenience in FIG. 5, but may include a plurality of layers.

The acoustic lens 158 is arranged outside the acoustic matching layer 157.
The acoustic lens 158 reduces friction with the stomach wall or the like against which the probe 150 is pressed, and also reduces a beam diameter in the Y axis direction of a beam transmitted from the ultrasonic transducer array 155. This configuration enables vivid display of an ultrasonic image. The acoustic lens 158 is formed of, for example, a silicon-based resin, a butadiene-based resin, or a polyurethane-based resin, but may be formed by further containing powder of oxidized titanium, alumina, silica, or the like. A value of acoustic impedance of the acoustic lens 158 can be within a range between the value of acoustic impedance of the acoustic matching layer 157 and the value of acoustic impedance of the subject.

The biopsy needle 410 includes a sheath portion 411, and a needle portion 412 that is inserted through the inside of the sheath portion 411. The sheath portion 411 includes, for example, a coil-shaped sheath, and has flexibility. The length of the sheath portion 411 can be adjusted as appropriate according to the length of the insertion portion 110. The needle portion 412 is formed of, for example, a nickel-titanium alloy or the like, and the distal end thereof is processed to be sharp. This allows the needle portion 412 to be inserted into a hard lesion. In addition, surface processing such as sandblast processing and dimple processing may be performed on the surface of the needle portion 412. With this configuration, it becomes possible to further reflect ultrasonic waves on the surface of the needle portion 412. This allows the needle portion 412 to be clearly displayed in the ultrasonic image, which will be described later.

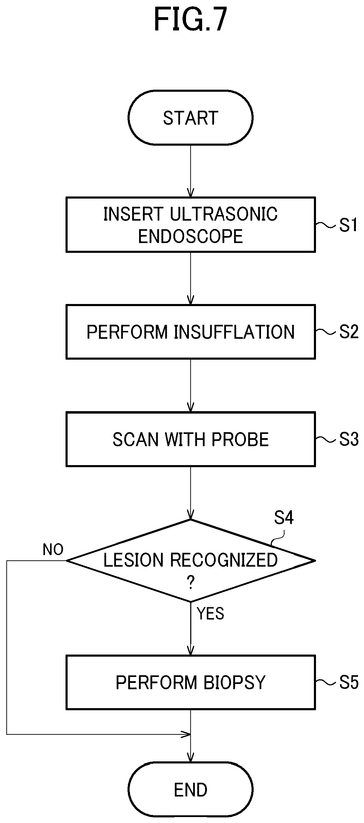

Although not illustrated, various configurations of the needle portion 412 have been proposed, and any configurations may be applied to the biopsy needle 410 that is used in the endoscope system 1 according to the present embodiment. For example, the needle portion 412 includes a cylinder needle and a stylet that is inserted through the inside of a cylinder of the needle. At least one of a distal end of the needle or a distal end of the stylet has a sharp shape, but, for example, one needle may constitute the needle portion 412. Note that the needle may be referred to as an outer needle or the like. In addition, the stylet may be referred to as an inner needle or the like. In any configurations of the needle portion 412, it is possible to make a predetermined space for collecting cellular tissues regarding the lesion. The cellular tissues regarding the lesion are taken into the predetermined space by, for example, a biopsy (step S5), which will be described later with reference to FIG. 7.

For example, the user inserts the biopsy needle 410 from the insertion opening 190 in a state where the needle portion 412 is housed in the sheath portion 411. As the user inserts the biopsy needle 410, a first stopper mechanism that is located at a predetermined position on the base end side of the distal end opening portion 134 comes into contact with the distal end of the sheath portion 411. The first stopper mechanism is not illustrated. This prevents the sheath portion 411 from moving from the predetermined position toward the distal end side. Alternatively, a mechanism for stopping the advance of the sheath portion 411 may be arranged on the base end side of the insertion opening 190 and serve as the first stopper mechanism. With this state of the sheath portion 411, the user uses a first slider, which is not illustrated, to project only the needle portion 412 from the distal end side of the sheath portion 411. In a case where the needle portion 412 includes the needle and the stylet, the user may be able to project the needle portion 412 in a state where the needle and the stylet are integrated with each other. With this configuration, as illustrated in FIG. 4, the needle portion 412 projects from the distal end opening portion 134. Alternatively, for example, only the stylet may be retreated with use of a second slider without a change of the position of the needle. The second slider is not illustrated. This enables formation of a space between the needle and the stylet. The space will be described later.

Note that the above-mentioned slider mechanism of the needle portion may further include a second stopper mechanism so as to be capable of adjusting a maximum stroke amount of the needle portion 412. The maximum stroke amount of the needle portion 412 is a maximum projectable length of the needle portion 412 from the sheath portion 411. This can prevent the needle portion 412 from excessively projecting from the sheath portion 411.

In work of the biopsy (step S5), which will be described later with reference to FIG. 7, each portion that constitutes the biopsy needle 410 can be manually advanced/retreated by the user, but the biopsy needle 410 may be capable of electrically advancing/retreating, which will be described in detail later with reference to FIG. 21 or the like.

The user uses the ultrasonic endoscope 100 including the distal end portion 130 having the above-mentioned configuration, whereby the endoscope system 1 acquires the ultrasonic image. While the ultrasonic image in a brightness mode (B mode) will be given as an example in the following description, this does not prevent the endoscope system 1 of the present embodiment from being capable of further displaying the ultrasonic image in another mode. The other mode is, for example, an amplitude mode (A mode), a coronal mode (C mode), a motion mode (M mode), or the like.

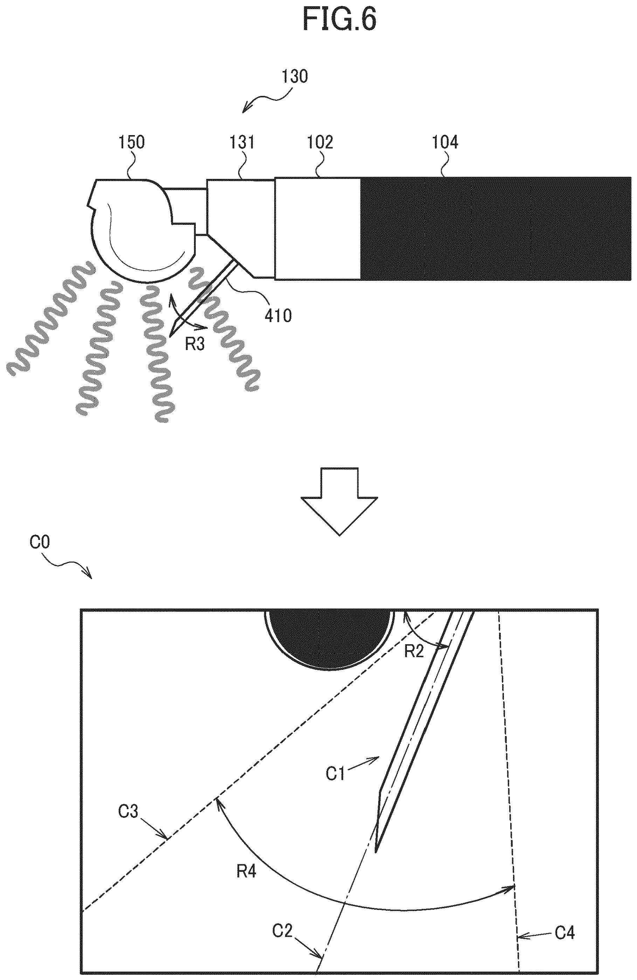

The B mode is a display mode for converting amplitude of ultrasonic waves to luminance and display a tomographic image. An upper center portion of the ultrasonic image is a region corresponding to the probe 150. For example, as illustrated in an upper stage of FIG. 6, scan is performed with ultrasonic waves in a scan range along a curved surface of the probe 150, for example, in a range at a predetermined distance from the center of curvature of the curved surface. In a case where the projecting biopsy needle 410 is included in the scan range, for example, an ultrasonic image is drawn so as to include an image corresponding to the biopsy needle 410 as indicated by C0.

While an actual ultrasonic image is a grayscale image, FIG. 6 schematically illustrates an image corresponding to the biopsy needle 410 and an image regarding region marker information, which will be described later, and other images are omitted for descriptive convenience. The ultrasonic image in FIG. 6 is displayed so that the left side of the image is the distal end side, the right side of the image is the base end side, and the upper center of the image is the curved surface of the probe 150. The same applies to ultrasonic images which are subsequently illustrated.

Since the longitudinal direction of the biopsy needle 410 is not matched with the longitudinal direction of the distal end portion 130 as described above with reference to FIG. 5, the image corresponding to the biopsy needle 410 is displayed in the ultrasonic image so as to be inclined by an angle indicated by R2 from the right side on the upper side of the ultrasonic image. The angle indicated by R2 in FIG. 6 corresponds to the angle indicated by R1 and described with reference to FIG. 5, that is, the angle of the biopsy needle 410. In other words, the angle indicated by R2 in FIG. 6 is the angle of the biopsy needle 410 on the ultrasonic image. Since the image processing is performed, the angle indicated by R2 in FIG. 6 is not necessarily matched with the inclination angle of the raising base 135 indicated by R1 in FIG. 5. To address this, for example, a second table indicating a correspondence relationship between the angle indicated by R1 in FIG. 5 and the angle indicated by R2 in FIG. 6 is stored in the memory 12, and a method of converting the angle of the biopsy needle 410 indicated by R2 on the ultrasonic image using the second table is used, whereby the angle of the biopsy needle 410 on the ultrasonic image can be handled as the actual angle of the biopsy needle 410.

In the present embodiment, for example, a range in which the biopsy needle 410 can be drawn may be shown on the ultrasonic image. A structure of each portion constituting the distal end portion 130 and a range of the inclination angle of the raising base 135 are determined by design as indicated by R3 in Fig.6. In addition, a positional relationship between the probe 150 and the distal end opening portion 134 is fixed. Hence, a range of a region in which the biopsy needle 410 is displayed on the ultrasonic image can be preliminarily calculated as indicated by R4 in Fig.6. Thus, a movable range image indicating the range is preliminarily stored in the memory 12, and image processing to perform display so as to superimpose the ultrasonic image acquired from the ultrasonic endoscope 100 and the movable range image on each other is performed, whereby display of the movable range image as indicated by C3 and C4 in FIG. 6 can be implemented. An interval between a dotted line indicated by C3 and a dotted line indicated by C4 in FIG. 6 is a range in which the biopsy needle 410 can be displayed on the ultrasonic image. In other words, the movable range image is an image that indicates a contour of a region made of a set of images of the biopsy needle 410 that can be displayed on the ultrasonic image.

Note that the endoscope system 1 may be capable of adjusting a display position of the movable range image. With this configuration, a predetermined error is corrected, and the movable range of the biopsy needle 410 corresponding to the movable range image and the actual movable range of the biopsy needle 410 can be matched with each other with high accuracy. The predetermined error is, for example, an error based on a tolerance in processing of the distal end portion 130, an error based on how the sheath portion 411 of the biopsy needle 410 is curved, or the like. For example, the following method can implement adjustment of the display position of the movable range image.

For example, the user uses a drawing function of a touch panel or another function to perform drawing or the like of a straight line so as to be superimposed on a displayed image of the biopsy needle 410 as indicated by C1 in Fig.6, whereby the endoscope system 1 acquires information of a first straight line based on coordinates on the ultrasonic image as indicated by C2 in FIG. 6. The endoscope system 1 then compares the angle of the biopsy needle 410 that is obtained from an inclination of the first straight line and the above-mentioned second table and the angle of the biopsy needle 410 that is grasped based on the above-mentioned angle sensor or the raising base operation wire 136, and performs processing of complementing a value of the second table so that these angles are matched with other. This configuration enables more accurate display of the movable range image. Note that the first straight line indicated by C2 may be displayable as an image. In a case where the inclination angle of the raising base 135 is adjusted while the image of the first straight line is displayed on the ultrasonic image, the image of the first straight line may be rotationally moved in conjunction with the adjustment. In the following description, assume that the movable range image is displayed accurately.

FIG. 7 is a flowchart describing an example of a manipulation using the endoscope system 1 of the present embodiment. The manipulation regarding the flow in FIG. 7 is also referred to as Endoscopic UltraSound-guided Fine Needle Aspiration (EUS-FNA). The EUS-FNA is an abbreviation for Endoscopic UltraSound-guided Fine Needle Aspiration. The flow in FIG. 7 is on the assumption that each device of the ultrasonic endoscope 100 is manually operated by the user, but each device of the ultrasonic endoscope 100 may be operated by, for example, the motorized endoscope system 1, which will be described later. The motorization mentioned herein means that a medical device is driven by an actuator or the like based on an electric signal for controlling the operation of the device. More specifically, for example, at least one of advance/retreat of the insertion portion 110 or the like of the ultrasonic endoscope 100, curving of the curved portion 102, or roll rotation may be performed by electric driving. The same applies to advance/retreat, roll rotation, or the like of the treatment tool 400 represented by the biopsy needle 410.

First, the user inserts the ultrasonic endoscope 100 (step S1). Specifically, for example, the user inserts the insertion portion 110 to, for example, a predetermined part. The predetermined part is the stomach, the duodenum, or the like, and may be determined as appropriate depending on a part as an examination target. Although illustration or the like is omitted, for example, step S1 may be implemented by insertion of the insertion portion 110 and the overtube together into the predetermined part by the user in a state where the insertion portion 110 is inserted into the overtube. This allows another treatment tool 400 other than the insertion portion 110 to be inserted into the overtube.

Thereafter, the user performs insufflation (step S2). Specifically, for example, the user connects the ultrasonic endoscope 100 to an insufflation device, which is not illustrated, and supplies predetermined gas into the predetermined part. The predetermined gas is, for example, the air, but may be carbon dioxide. The air mentioned herein is gas having a component ratio that is equivalent to that of the atmospheric air. Since the carbon dioxide is quickly absorbed into the living body in comparison with the air, a burden on the subject after the manipulation can be reduced. The predetermined gas is, for example, supplied from an air supply nozzle in the distal end portion 130, but may be supplied from, for example, an air supply tube inserted into the above-mentioned overtube. The air supply nozzle is not illustrated. Although details will be described later, the contracted stomach wall or the like is extended or the like by the supplied, predetermined gas and becomes in a state appropriate for an examination using the ultrasonic endoscope 100. Details about the state appropriate for the examination using the ultrasonic endoscope 100 will be described later.

Thereafter, the user performs scan with the probe 150 (step S3). For example, the user brings the probe 150 into contact with the stomach wall or the like, and causes the probe 150 to transmit ultrasonic waves toward an observation target part. The probe 150 receives reflected waves of the ultrasonic waves, and the endoscope system 1 generates an ultrasonic image based on the received signals. The user moves the probe 150 within a predetermined range while maintaining this state, and checks the presence/absence of the lesion in the observation target part.

Thereafter, the user determines whether or not he/she can recognize the lesion (step S4). Specifically, the user determines whether or not the lesion exists in the observation target part from luminance information or the like of the ultrasonic image obtained in step S3. In a case where the user cannot recognize the lesion (NO in step S4), he/she ends the flow. In contrast, in a case where the user can recognize the lesion (YES in step S4), he/she performs the biopsy (step S5). In the following description, a case where the biopsy needle 410 is used in a fine-needle aspiration biopsy that performs collection by aspiration is given as an example, but the biopsy needle 410 is not prevented from being used in another biopsy.

In the biopsy (step S5), the user performs an aspiration biopsy depending on a type of the biopsy needle 410. Note that the following description is not applied to all types of biopsy needles 410, and is given merely as an example. Although not illustrated, for example, the user moves the needle portion 412 in which a needle and a stylet are integrated with each other toward the distal end side until the needle portion 412 is sufficiently inserted into a cellular tissue regarding the lesion while observing the ultrasonic image. The user performs an operation of pulling only the stylet toward the base end side in a state where the needle portion 412 is inserted into the cellular tissue, and thereby forms a predetermined space and creates a negative pressure state. With this operation, the cellular tissue is sucked into the predetermined space. Thereafter, the user pulls the whole biopsy needle 410 toward the base end side, and can thereby collect an amount of the cellular tissue.

The endoscope system 1 of the present embodiment is capable of acquiring the image in which the region marker information of the lesion is set in the biopsy (step S5). For example, the user observes an ultrasound image indicated by C10, and detects the lesion within a range that is predicted to be the movable range in the vicinity of an image corresponding to the biopsy needle 410 indicated by C11. The user then uses the drawing function of the touch panel or another function to draw a landmark indicated by C12 so that the landmark corresponds to the region indicating the lesion. That is, the endoscope system 1 acquires each coordinate information included in the region corresponding to the landmark via a sensor of the touch panel or the like as the region marker information.

Additionally, the endoscope system 1 of the present embodiment calculates the angle of the biopsy needle 410 based on the region marker information of the lesion. For example, the calculation of the angle of the biopsy needle 410 can be implemented with use of the following method, but may be implemented by another method. First, the endoscope system 1 calculates a specific position for inserting the biopsy needle 410 on the landmark. The specific position is, for example, the centroid of the landmark, but may be the center, an outside edge, or the like, or a position instructed by the user with the touch panel or the like. Assume that the endoscope system 1 calculates a position of a mark indicated by C21 as the specific position in an ultrasonic image indicated by C20.

The endoscope system 1 then calculates the angle of the biopsy needle 410 for inserting the biopsy needle 410 into the lesion. As described above, for example, since the movable range image corresponds to a region predetermined by design, when the angle of the biopsy needle 410 is determined, an aggregation of coordinates included in the image of the biopsy needle 410 displayed on the ultrasonic image is unambiguously determined. Hence, for example, the endoscope system 1 performs processing of referring to a third table that associates the angle of the biopsy needle 410 and coordinates of the image of the biopsy needle 410 with each other and searching for the angle of the biopsy needle 410 corresponding to coordinates of the specific position. With this processing, for example, the endoscope system 1 calculates a second straight line passing the specific position as indicated by C23 and the angle of the biopsy needle 410 indicated by R22 based on the second straight line.

As described above, the endoscope system 1 of the present embodiment includes the processor 10. The processor 10 acquires the image in which the region marker information of the lesion is set with respect to the ultrasonic image of the ultrasonic endoscope 100 with the biopsy needle 410, and calculates the angle of the biopsy needle 410 for inserting the biopsy needle 410 into the lesion based on the movable range of the biopsy needle 410 and the region marker information.

In the biopsy (step S5) or the like, the biopsy needle 410 may be infallibly inserted into the lesion at a desired position. However, to actually insert the biopsy needle 410 at the desired position while watching the ultrasonic image, the user needs to pay attention such as adjustment of the angle of the biopsy needle 410 in consideration of the lesion being within the movable range of the biopsy needle 410, and may be thereby required to have a proficiency.

In this regard, the endoscope system 1 of the present embodiment acquires the image in which the region marker information of the lesion is set, and is thereby capable of visually grasping the lesion on the ultrasonic image. Additionally, the endoscope system 1 calculates the angle of the biopsy needle 410 based on the movable range of the biopsy needle 410 and the region marker information, and is thereby capable of directing the biopsy needle 410 to the desired position on the assumption that it can be confirmed that the position at which the biopsy needle 410 is desired to be inserted is within the movable range of the biopsy needle 410. This allows the user to easily perform work of inserting the biopsy needle 410 while watching the ultrasonic image. The specification of U.S. Unexamined Patent Application Publication No. 2019/0247127 discloses the method of marking the lesion or the like, but does not disclose that support is given to determine at which angle the biopsy needle 410 is adjusted in consideration of the movable range of the biopsy needle 410.

Alternatively, the method according to the present embodiment may be implemented as a calculation method. That is, the calculation method according to the present embodiment is to acquire the image in which the region marker information of the lesion is set with respect to the ultrasonic image of the ultrasonic endoscope 100 with the biopsy needle 410, and calculate the angle of the biopsy needle 410 for inserting the biopsy needle 410 into the lesion based on the movable range of the biopsy needle 410 and the region marker information. This enables obtaining of an effect that is similar to the above-mentioned effect.

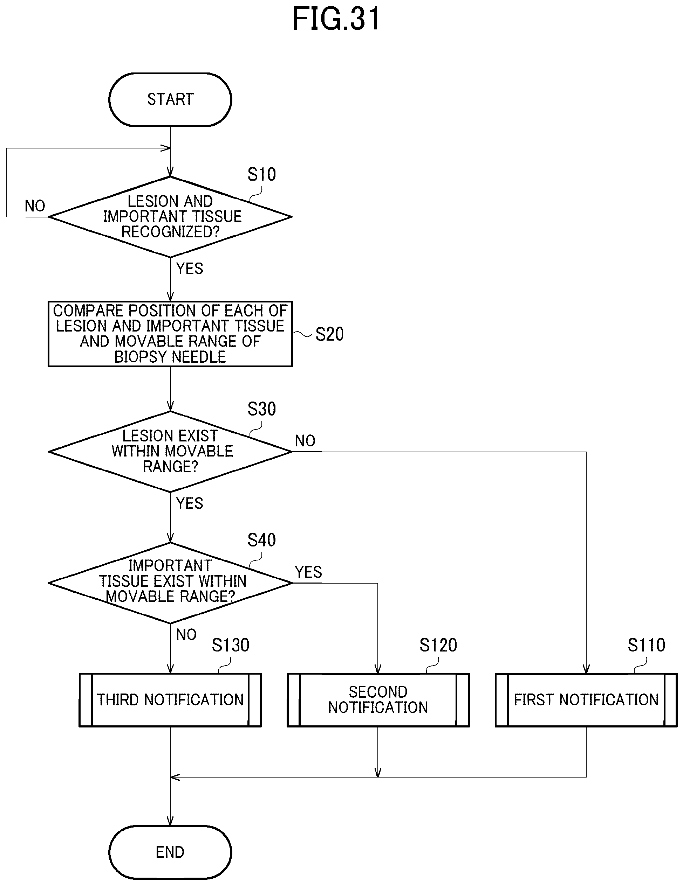

Note that the endoscope system 1 may display the mark indicated by C21 and the second straight line indicated by C23 as images, and display the images as operation support information for the user. Alternatively, the endoscope system 1 may display a first straight line indicated by C22 as an image, and display an instruction for matching the image of the first straight line with the image of the second straight line as the operation support information. That is, in the endoscope system 1 of the present embodiment, the processor 10 performs presentation processing for presenting the operation support information of the ultrasonic endoscope 100 to the user based on the calculated angle of the biopsy needle 410. This allows the user to easily adjust the angle of the biopsy needle 410 in comparison with a case where he/she observes only the ultrasonic image. Accordingly, the user can easily direct the biopsy needle 410 to the desired position of the lesion. Note that the operation support information of the present embodiment is not limited thereto, and details will be described later with reference to FIG. 31 or the like.

The above description has been given of the method of calculating only the angle of the biopsy needle 410, but the method according to the present embodiment is not limited thereto and may further enable calculation of the depth of the biopsy needle 410. The depth of the biopsy needle 410 is, for example, a projectable length of the needle portion 412 from the distal end of the sheath portion 411. As described above, because the position of the distal end of the sheath portion 411 and the maximum stroke amount of the needle portion 412 are known or for other reasons, the maximum length of the needle portion 412 displayed on the ultrasonic image based on the maximum stroke amount of the needle portion 412 can also be calculated together similarly to the case of calculation of the angle of the biopsy needle 410. With this configuration, for example, a movable range image indicated by C31 is displayed as part of an arc-shaped figure in an ultrasonic image indicated by C30. Note that the center of the arc is not displayed on the ultrasonic image. This is because the center of the arc corresponds to the position of the distal end opening portion 134, and ultrasonic waves do not reach the position.

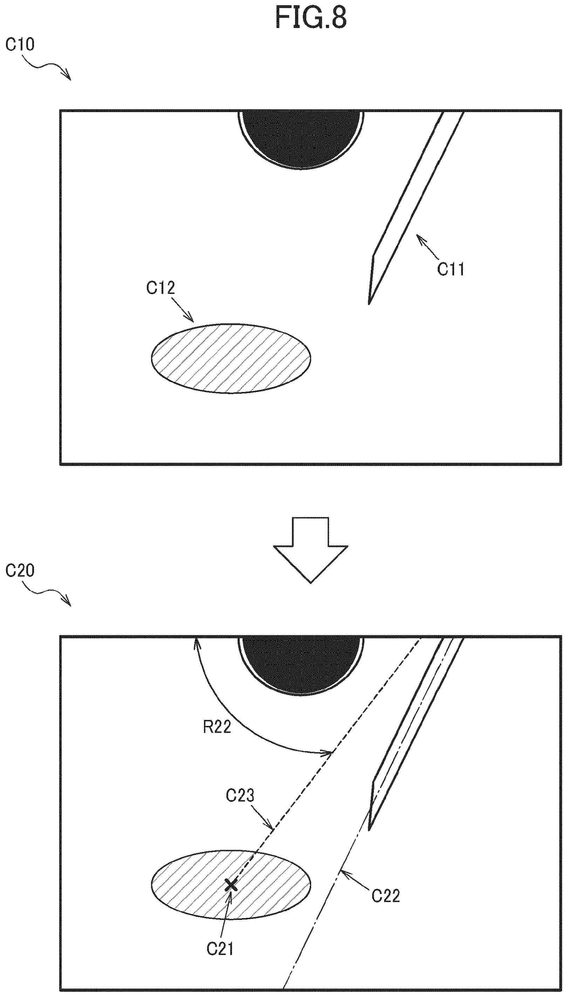

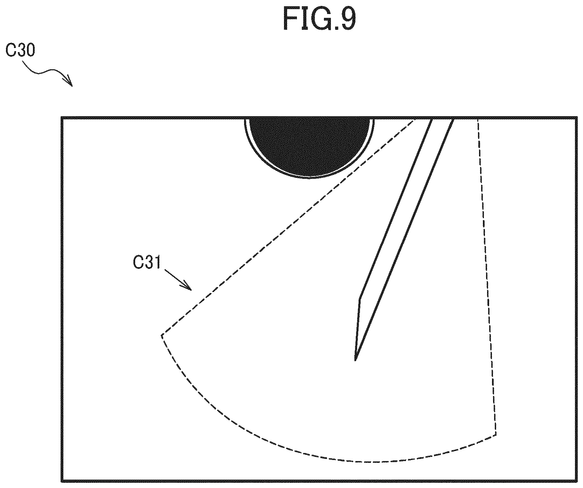

Although not illustrated, for example, the user observes the ultrasonic image, confirms that the lesion exists inside the movable range image indicated by C31 in FIG. 9, and thereafter applies the method described above with reference to FIG. 8 to obtain the above-mentioned specific position. The endoscope system 1 then performs processing of selecting the angle of biopsy needle 410 corresponding to the first straight line from the above-mentioned third table, processing of obtaining a length of the first straight line from coordinates of the specific position, and processing of obtaining a stroke length of the needle portion 412 for inserting the biopsy needle 410 into the lesion based on the length of the first straight line. Therefore, in the endoscope system 1 of the present embodiment, the processor 10 calculates the angle and depth of the biopsy needle 410 for inserting the biopsy needle 410 into the lesion based on the movable range of the biopsy needle 410 and the region marker information. This can create a state where the biopsy needle 410 is directed to the desired position and the depth for inserting the biopsy needle 410 can be grasped. This allows the user to easily perform work of operating the above-mentioned needle or the like while watching the ultrasonic image and inserting the biopsy needle 410 in appropriate depth.

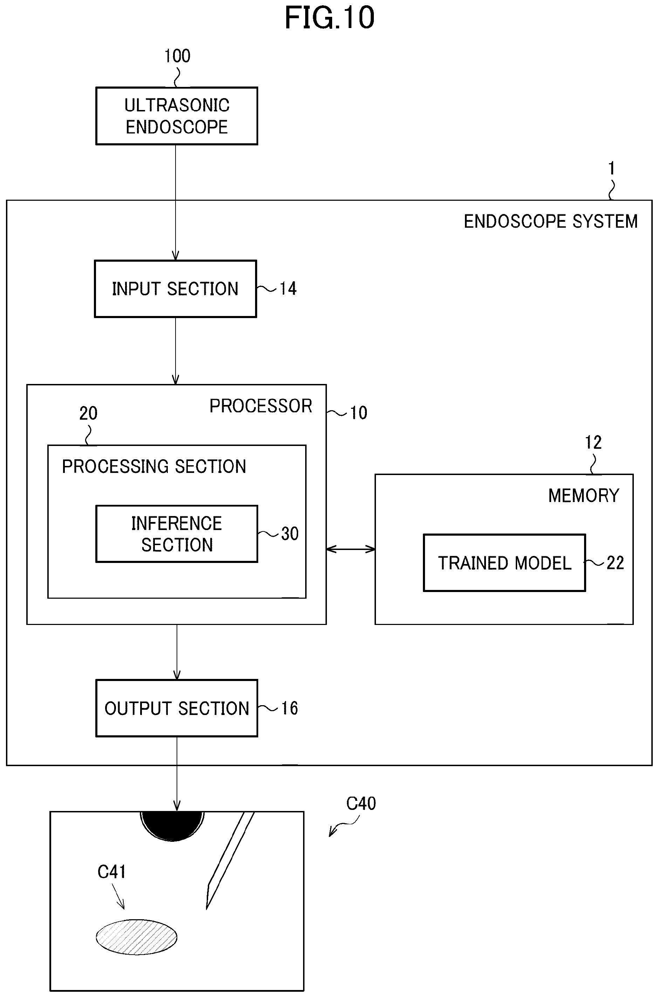

While the above description has been given of the example in which the endoscope system 1 acquires the region marker information by the user's input work, the method according to the present embodiment is not limited thereto. For example, the endoscope system 1 may be capable of acquiring the region marker information using the trained model 22 described above with reference to FIG. 2. Specifically, for example, the endoscope system 1 of the present embodiment is configured as a configuration example illustrated in FIG. 10, and can thereby implement detection of the region marker information using the trained model 22. In FIG. 10, the trained model 22 is used for the endoscope system 1 including the memory 12 that stores the trained model 22, an input section 14, the processor 10 including the processing section 20, and an output section 16.

In FIG. 10, the processing section 20 includes an inference section 30. Specifically, for example, the processing section 20 reads out the trained model 22 from the memory 12, executes the program regarding the trained model 22, and thereby functions as the inference section 30.

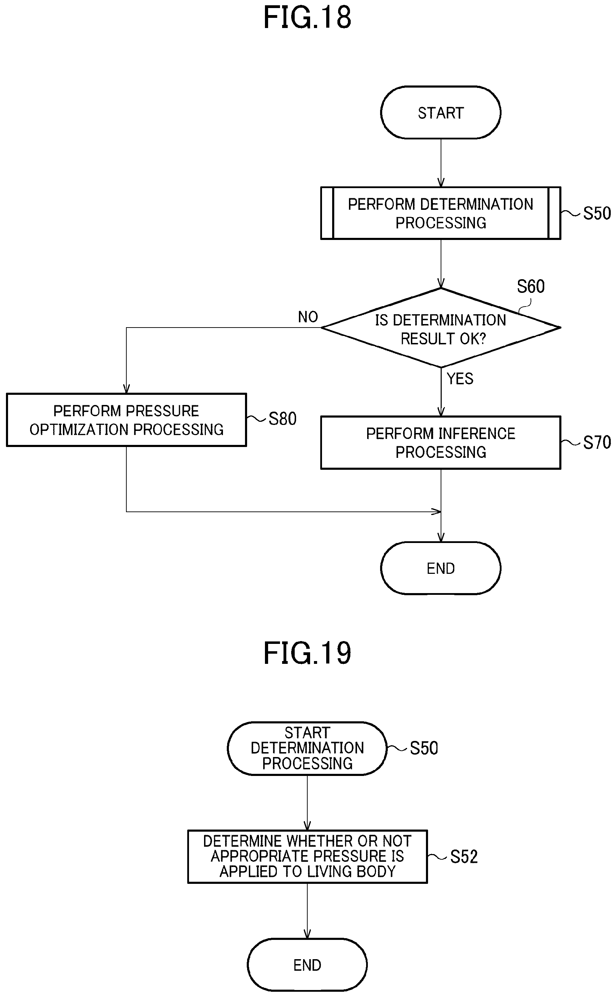

The input section 14 is an interface that receives input data from the outside. Specifically, the input section 14 is an image data interface that receives the ultrasonic image as a processing target image. For example, the input section 14 uses the received ultrasonic image as the input data to the trained model 22 and the inference section 30 performs inference processing (step S70) or the like, whereby a function as the input section 14 is implemented. The inference processing will be described later with reference to FIG. 18.

The output section 16 is an interface that transmits data estimated by the inference section 30 to the outside. For example, the output section 16 outputs output data from the trained model 22 as an ultrasonic image indicated by C40 in FIG. 10, whereby a function as the output section 16 is implemented. The ultrasonic image indicated by C40 is displayed so that region marker information indicated by C41 is superimposed thereon. That is, unlike the case described with reference to FIG. 8, the region marker information indicated by C41 is automatically displayed without the user's input work. An output destination of the output data is, for example, a predetermined display device connected to the endoscope system 1. For example, the output section 16 serves as an interface that can be connected to the predetermined display device, whereby an image that is obtained by superimposing the ultrasonic image and the region marker information on each other is displayed on the predetermined display device, and a function as the output section 16 is implemented. As described later with reference to FIG. 21 or the like, in a case where the endoscope system 1 is motorized, a display device 900 corresponds to the predetermined display device.

As an acquisition method for acquiring the region marker information, it is possible to use, for example, a method of segmenting the ultrasonic image into a plurality of regions by semantic segmentation and using a region from which the lesion can be read based on a result of the segmentation as the region marker information, or another method. For example, a bounding box with which the lesion or the like that can be read from the ultrasonic image by object detection may be the region marker information.

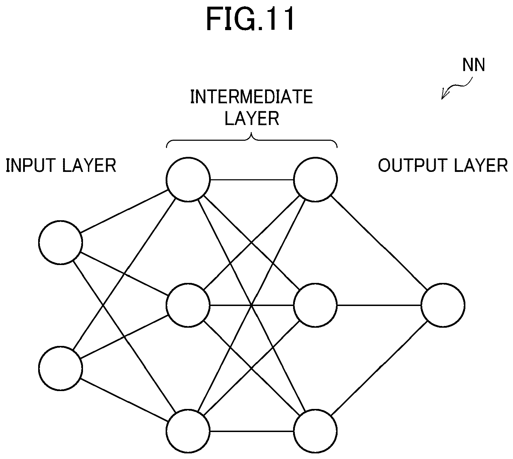

In the trained model 22 of the present embodiment, a neural network NN is included in at least part of the model. The neural network NN includes, as illustrated in FIG. 11, an input layer that takes input data, an intermediate layer that executes calculation based on an output from the input layer, and an output layer that outputs data based on an output from the intermediate layer. While FIG. 11 exemplifies a network including the intermediate layer composed of two layers, the intermediate layer may include one layer, or three or more layers. In addition, the number of nodes included in each layer is not limited to that in the example of FIG. 11. As illustrated in FIG. 11, a node included in a given layer is connected to a node in an adjacent layer. A weight coefficient is assigned between connected nodes. Each node multiplies an output from a node in a former stage by the weight coefficient and obtains a total value of results of multiplication. Furthermore, each node adds a bias to the total value and applies an activation function to a result of addition to obtain an output from the node. This processing is sequentially executed from the input layer to the output layer, whereby an output from the neural network NN is obtained. As the activation function, various functions such as a sigmoid function and a rectified linear unit (ReLU) function are known, and a wide range of these functions can be applied to the present embodiment.

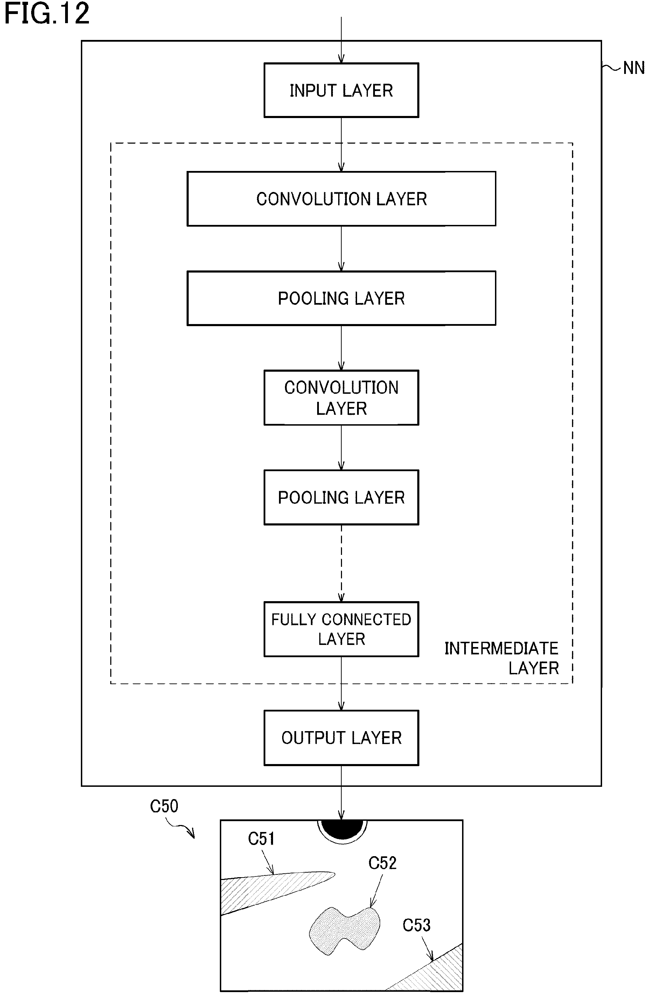

Models having various configurations of the neural network NN have been known, and a wide range of these models are applicable to the present embodiment. The neural network NN may be, for example, a convolutional neural network (CNN) used in the field of image recognition, or another model such as a recurrent neural network (RNN). In the CNN, for example, as illustrated in FIG. 12, the intermediate layer includes a plurality of sets each including a convolution layer and a pooling layer, and a fully connected layer. The convolution layer executes convolution calculation using a filter on a near node in the former layer, executes feature extraction such as edge extraction from the ultrasonic image as the input data, and acquires a feature map. The pooling layer reduces in size the feature map output from the convolution layer into a new feature map, and provides the extracted feature with robustness. The fully connected layer connects all of nodes in an immediately former layer. With use of the trained model 22 including such a model, an ultrasonic image indicated by C50 is eventually output. A region indicated by C51, a region indicated by C52, and a region indicated by C53 are detected by segmentation in the ultrasonic image indicated by C50, and are displayed as the region marker information as being superimposed on the ultrasonic image.

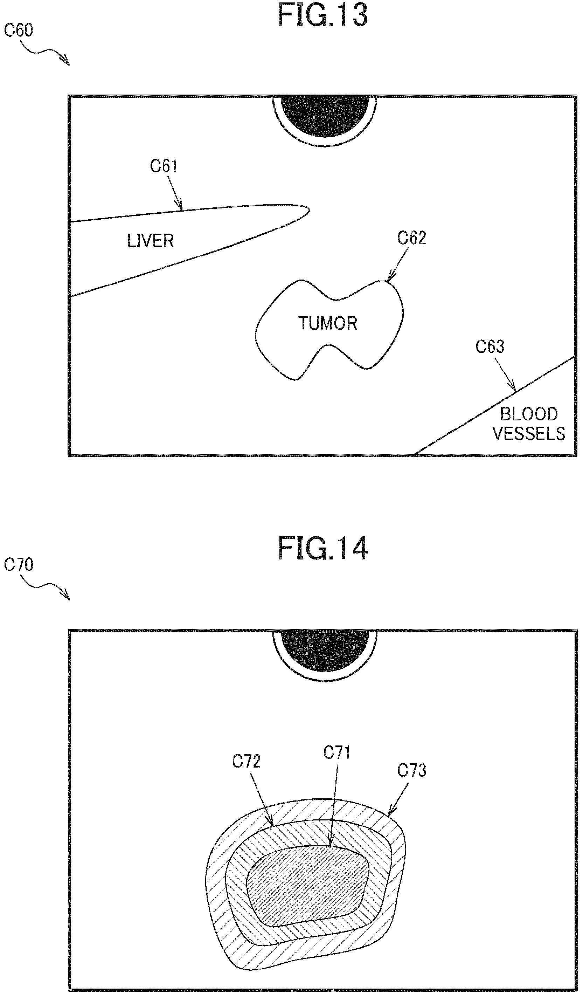

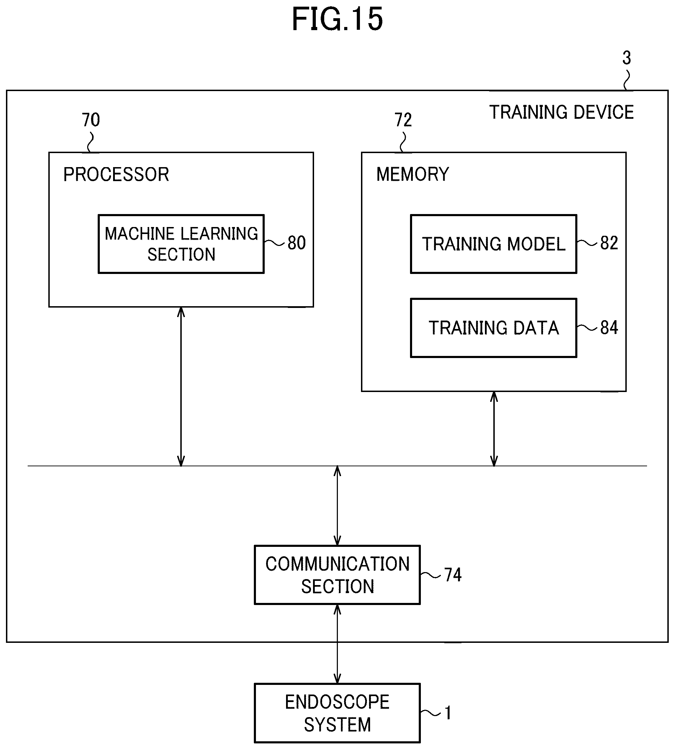

In the ultrasonic image indicated by C50 in FIG. 12, the region indicated by C51, the region indicated by C52, and the region indicated by C53 are displayed so that segmentation has been performed in respective different colors, but an example of output images is not limited thereto. For example, instead of the ultrasonic image indicated by C50 in FIG. 12, an ultrasonic image indicated by C60 in FIG. 13 may be output. In the ultrasonic image indicated by C60, a region indicated by C61, a region indicated by C62, and a region indicated by C63 are subjected to segmentation, and are provided with respective names. These names are, for example, names of classes associated with output data, which will be described later with reference to FIG. 15.

Alternatively, for example, an ultrasonic image indicated by C70 in FIG. 14 may be output. In the ultrasonic image indicated by C70, regions after segmentation are displayed in a contour-line pattern. With this configuration, features of the detected regions can be indicated in a stepwise manner. For example, the region indicated by C71 is a region with an extremely high possibility of being a tumor; the region indicated by C72 is a region indicating a middle possibility of being the tumor; and the region indicated by C73 is a region that is suspected to be the tumor.

In addition, the neural network NN according to the present embodiment may be a model developed further from the CNN. Examples of a model for segmentation include a Segmentation Network (SegNet), a Fully Convolutional Network (FCN), and a U-Shaped Network (U-Net), and a Pyramid Scene Parsing Network (PSPNet). In addition, examples of a model for object detection include a You Only Look Once (YOLO) and a Single Shot Multi-Box Detector (SSD). Although details of these models are not illustrated, the intermediate layer of the neural network NN is modified according to these models. For example, convolution layers may be continuous in the intermediate layer, or the intermediate layer may further include another layer. The other layer is a reverse pooling layer, a transposed convolution layer, or the like. With adoption of these models, the accuracy of segmentation can be increased.

Machine learning on the trained model 22 is performed by, for example, a training device 3. FIG. 15 illustrates a block diagram illustrating a configuration example of the training device 3. The training device 3 includes, for example, a processor 70, a memory 72, and a communication section 74, and the processor 70 includes a machine learning section 80.

The communication section 74 is a communication interface that is capable of communicating with the endoscope system 1 in a predetermined communication method. The predetermined communication method is, for example, a communication method in conformity with a wireless communication standard such as Wireless Fidelity (Wi-Fi) (registered trademark), but may be a communication method in conformity with a wired communication standard such as a universal serial bus (USB). With this configuration, the training device 3 transmits the trained model 22 subjected to the machine learning to the endoscope system 1, and the endoscope system 1 is thereby capable of updating the trained model 22. Note that FIG. 15 illustrates the example in which the training device 3 and the endoscope system 1 are separately arranged, but this does not prevent adoption of a configuration example in which the endoscope system 1 includes a training server corresponding to the training device 3.

The processor 70 performs control to input/output data to/from functional sections such as the memory 72 and the communication section 74. The processor 70 can be implemented by hardware or the like similar to that of the processor 10 described with reference to FIG. 1. The processor 70 executes various kinds of calculation processing based on a predetermined program read out from the memory 72, an operation input signal from an operation section, or the like, and controls an operation of outputting data to the endoscope system 1, and the like. The operation section is not illustrated in FIG. 15. The predetermined program mentioned herein includes a machine learning program, which is not illustrated. That is, the processor 70 reads out the machine learning program, necessary data, and the like from the memory 12 as appropriate and executes the machine learning program, necessary data, and the like, and thereby functions as a machine learning section 80.

In addition to the machine learning program, which is not illustrated, a training model 82 and training data 84 are stored in the memory 72. The memory 72 can be implemented by a semiconductor memory or the like similar to that of the memory 12. The training data 84 is, for example, an ultrasonic image, but may include another data, details of which will be described later when need arises. Ultrasonic images, as the training data 84, corresponding to the number of types of subjects that can be input data are stored in the memory 72.

Specifically, the training device 3 inputs input data out of the training data 84 to the training model 82 and performs calculation in the forward direction according to a model configuration using a weight coefficient at this time to obtain an output. An error function is calculated based on the output and a correct label, and the weight coefficient is updated to make the error function smaller.

The output layer of the training model 82 includes, for example, N nodes. N is the number of types of regions that can be the region marker information. A first node is information indicating a probability that input data belongs to a first class. Similarly, an N-th node is information indicating a probability that input data belongs to an N-th class. The first to N-th classes include classes based on at least the important (predetermined) tissue and the lesion. The important tissue mentioned herein is a tissue that the biopsy needle 410 should be avoided from coming in contact with, for example, an organ such as the liver, the kidney, the pancreas, the spleen, and the gallbladder, blood vessels, or the like, and is considered to be in a normal state in appearance. The lesion mentioned herein is a portion that is considered to be in a state different in appearance from a normal state, and is not necessarily limited to a portion that attributes to a disease. That is, the lesion is, for example, a tumor, but is not limited thereto, and may be a polyp, an inflammation, a diverticulum, or the like. In addition, the lesion may be either a neoplastic lesion or a non-neoplastic lesion. In a case of the neoplastic lesion, the lesion may be either benign or malignant. In consideration of these matters, categories of the important tissue and the lesion are determined as appropriate.

With the training model 82 having the above-mentioned configuration, for example, in the case of semantic segmentation, when one dot in the ultrasonic image is input as input data, the liver as the first class is output as output data. When another one dot is input as input data, processing of outputting, as output data, a malignant tumor as the N-th class is performed the number of times corresponding to the number of dots constituting the ultrasonic image. As a result, the ultrasonic image that is segmented based on the liver, the malignant tumor, or the like is eventually output. With this configuration, the detection of the region marker information is implemented. Note that segmentation is not necessarily performed in all the classes. This is because there is no need for performing segmentation with respect to a tissue that the biopsy needle 410 has no problem of coming in contact with.

In this manner, the endoscope system 1 of the present embodiment includes the memory 12 that stores the trained model 22 trained to output, to the ultrasonic image captured by the ultrasonic endoscope 100, the region marker information as the detection target in the ultrasonic image, and the processor 10 detects the region marker information based on the ultrasonic image and the trained model 22. This configuration enables such automatic display as that the region marker information is superimposed on the ultrasonic image captured by the ultrasonic endoscope 100.

In addition, the endoscope system 1 of the present embodiment may detect the region marker information based on the ultrasonic image when an appropriate pressure that guarantees accuracy of the region marker information is applied to the living body and the trained model 22 trained as described above. For example, before actually performing the above-mentioned scan with the probe (step S3), the user confirms that the ultrasonic image is an ultrasonic image when the appropriate pressure is applied to the living body by the following method.

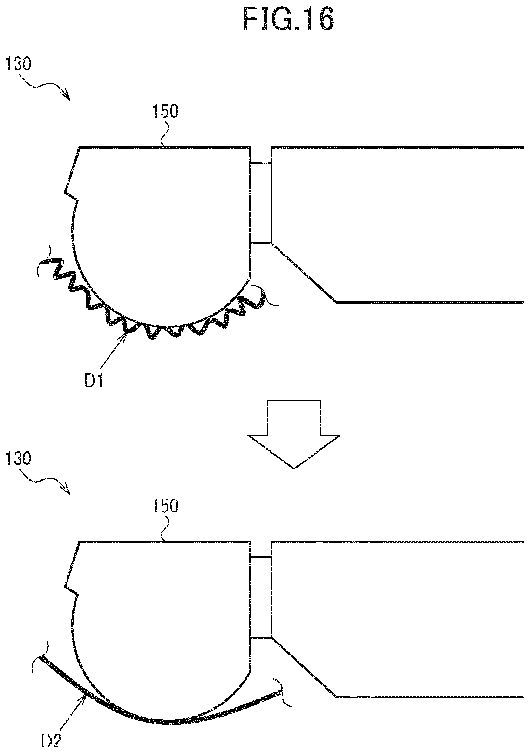

For example, since the stomach wall or the like is contracted from the beginning and folds and the like exist on the stomach wall as described above with reference to FIG. 7, there can occur a situation where the probe 150 of the ultrasonic endoscope 100 is not sufficiently pressed against the stomach wall in the scan with the probe (step S3) in FIG. 7, as indicated by D1 in FIG. 16. Under such a situation, a gap is generated between the probe 150 and the stomach wall, and ultrasonic waves transmitted from the probe 150 are reflected in the gap. Hence, there is a possibility that the probe 150 receives reflected waves that are different from reflected waves that are supposed to be obtained. As a result, there is a possibility that the endoscope system 1 acquires an ultrasonic image that is different from an ultrasonic image that is supposed to be obtained. In a case where such an ultrasonic image is input to the trained model 22, there is a possibility that detected region marker information is different from that in a case where the ultrasonic image that is supposed to be obtained is input to the trained model 22. In this manner, the accuracy of the region marker information is not guaranteed unless the probe 150 is sufficiently pressed against the stomach wall. The accuracy of the region marker information mentioned herein is a degree of a scale of variation between a true detection target region of the region marker information and the region that is actually detected as the region marker information. The scale mentioned herein is, for example, dispersion or the like. Specifically, for example, the accuracy of the region marker information of the lesion being not guaranteed means that a dispersion value regarding the variation between the true region of the lesion and the region that is actually detected as the region marker information of the lesion does not satisfy certain criteria.

Hence, the endoscope system 1 of the present embodiment determines whether or not a pressing pressure is appropriate by the method that will be described later. When the probe 150 is pressed against the stomach wall or the like with the appropriate pressing pressure, no gap is generated between the probe 150 and the stomach wall or the like as indicated by D2 in FIG. 16, whereby the endoscope system 1 is capable of acquiring an appropriate ultrasonic image. Consequently, the accuracy of the region marker information can be guaranteed.

Note that examples of a method of determining whether or not the pressing pressure of the probe 150 is appropriate include a method of arranging a first pressure sensor, which is not illustrated, at a predetermined position of the housing 151 and making determination based on a measured value from the first pressure sensor. The first pressure sensor can be implemented by, for example, a micro electro mechanical systems (MEMS) or the like. Alternatively, part of the plurality of ultrasonic transducers 156 that constitutes the ultrasonic transducer array 155 may be used as the first pressure sensor. This is because a piezoelectric element that constitutes the ultrasonic transducer 156 can also be used as the first pressure sensor.

In addition, the endoscope system 1 of the present embodiment may determine whether or not an intraluminal pressure is appropriate in the insufflation (step S2) described above with reference to FIG. 7. Examples of a method of determining whether or not the intraluminal pressure is appropriate include a method of arranging a second pressure sensor such as an MEMS sensor at a predetermined position of the distal end portion 130 and making determination based on a measured value form the second pressure sensor.

Alternatively, the endoscope system 1 may further use an in-vivo image captured by the imaging sensor in the distal end portion 130 to determine whether or not the intraluminal pressure is appropriate. For example, with supply of the above-mentioned gas, the stomach undergoes a first state where the contracted stomach swells, a second state where tension is applied so that the stomach swells and the stomach wall extends, and a third state where the stomach swells and the stomach wall becomes unable to extend further. The third state is considered to be a state that is appropriate for the probe 150 to come in contact with the stomach wall. Hence, the endoscope system 1, for example, captures an image of the inside of the lumen with the imaging sensor while supplying the predetermined gas, associates the captured in-vivo image and the measured value from the second pressure sensor with each other, and determines the measured value from the second pressure sensor, as the appropriate pressure at the time when it can be confirmed that the stomach is in the third state from the in-vivo image.

Alternatively, the endoscope system 1, for example, may observe the in-vivo image to determine whether or not the pressure is the appropriate pressure. For example, in the insufflation (step S2), the endoscope system 1 compares in-vivo images captured while supplying the predetermined gas, and thereby preliminarily acquires in-vivo images in the first state, the second state, and the third state, which are described above. In the scan with the probe (step S3), the user operates the probe 150 while confirming that the captured in-vivo image is the in-vivo image in the third state. Note that the in-vivo image thus acquired may be used for determination about whether or not the above-mentioned pressing pressure of the probe 150 is appropriate.

In this manner, the endoscope system 1 of the present embodiment includes the memory 12 that stores the trained model 22 trained to output, to the ultrasonic image captured by the ultrasonic endoscope 100, the region marker information as the detection target in the ultrasonic image, and the processor 10. The processor 10 outputs the region marker information detected based on the ultrasonic image when the appropriate pressure that guarantees the accuracy of the region marker information is applied to the living body and the trained model 22, as being superimposed on the ultrasonic image.

Since the ultrasonic image is an image based on an echo signal, the probe 150 is unable to receive an accurate echo unless the probe 150 is in intimate contact with the stomach wall, and there is a possibility that, for example, an ultrasonic image that is not displayed at a luminance corresponding to the lesion or the like in a portion in which the lesion or the like is supposed to exist is drawn. Hence, even if the above-mentioned method disclosed in the specification of U.S. Unexamined Patent Application Publication No. 2019/0247127 is applied, there is a possibility that the important tissue, the lesion, or the like is not marked with high accuracy.

In this regard, with the application of the method according to the present embodiment, the region marker information is detected with use of the ultrasonic image when the appropriate pressure is applied and the trained model 22, whereby the region marker information can be detected with high accuracy. This enables acquisition of the ultrasonic image on which the region marker information of the lesion or the like is superimposed more appropriately. The specification of U.S. Unexamined Patent Application Publication No. 2019/0247127 described above does not disclose that the application of the appropriate pressure guarantees the accuracy of detection of the region marker information.

Alternatively, the method according to the present embodiment may be implemented as an information output method. That is, the information output method according to the present embodiment is based on the trained model 22 trained to output, to the ultrasonic image captured by the ultrasonic endoscope 100, the region marker information as the detection target in the ultrasonic image. The trained model 22 outputs the region marker information detected based on the ultrasonic image when the appropriate pressure that guarantees the accuracy of the region marker information is applied to the living body and the trained model 22, as being superimposed on the ultrasonic image. This enables obtaining of an effect that is similar to the above-mentioned effect.

Alternatively, the appropriate pressure may be a pressure that is set based on the pressing pressure of the probe 150 of the ultrasonic endoscope 100. With this configuration, the endoscope system 1 is capable of acquiring the ultrasonic image when the appropriate pressure is applied to the living body based on the pressing pressure of the probe 150.

Alternatively, the appropriate pressure may be a pressure that is set based on the intraluminal pressure detected from the second pressure sensor as the pressure sensor. With this configuration, the endoscope system 1 is capable of acquiring the ultrasonic image when the appropriate pressure is applied to the living body based on the intraluminal pressure.

Additionally, the processor 10 may perform estimation processing based on the in-vivo image captured by the imaging sensor of the ultrasonic endoscope 100 to estimate whether the pressure is the appropriate pressure. With this configuration, the endoscope system 1 is capable of determining whether or not it has been able to acquire the ultrasonic image when the appropriate pressure is applied to the living body based on the in-vivo image.

Additionally, the region marker information may include marker information corresponding to the region of the lesion and marker information corresponding to the region of the important tissue. With this configuration, the endoscope system 1 is capable of detecting and displaying the region marker information corresponding to the region of the lesion and the region marker information corresponding to the region of important tissue with respect to the ultrasonic image as the input data. This allows the user to make determination about the operation of the biopsy needle 410 appropriately while watching the ultrasonic image.

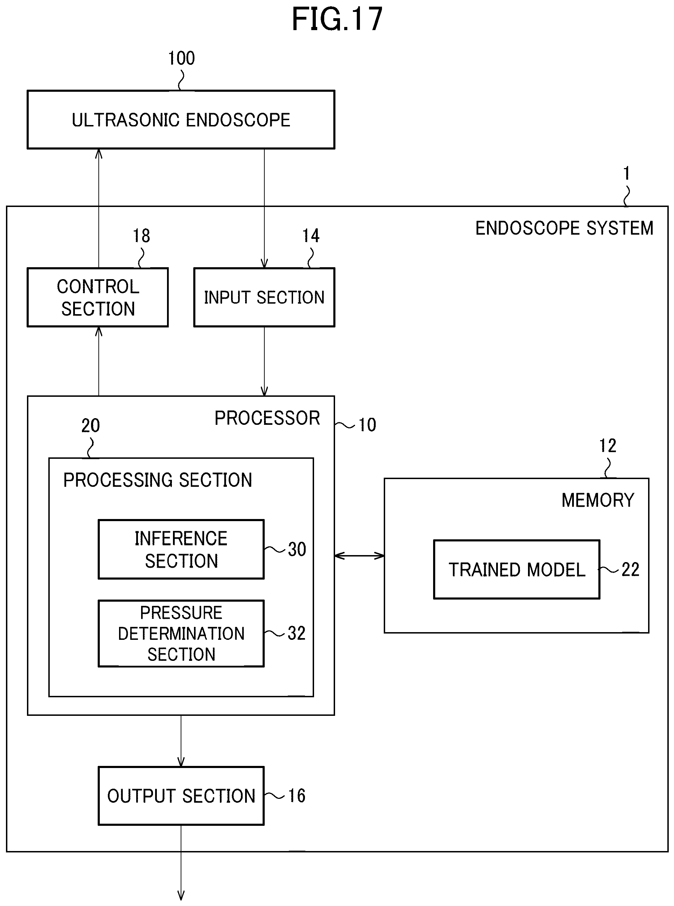

Alternatively, the endoscope system 1 of the present embodiment may acquire the ultrasonic image while determining whether or not the pressure is the appropriate pressure in real time. In addition, the endoscope system 1 may feedback the pressure being not the appropriate pressure to the user or the like. In this case, for example, the endoscope system 1 has a configuration in a configuration example illustrated in FIG. 17, and the determination about whether the pressure is the appropriate pressure can be implemented by, for example, execution of also processing in a processing example described in a flowchart in FIG. 18 together with the biopsy (step S5). The configuration example in FIG. 17 is different from the configuration example in FIG. 10 in that the endoscope system 1 further includes a control section 18 and the processing section 20 further includes a pressure determination section 32.

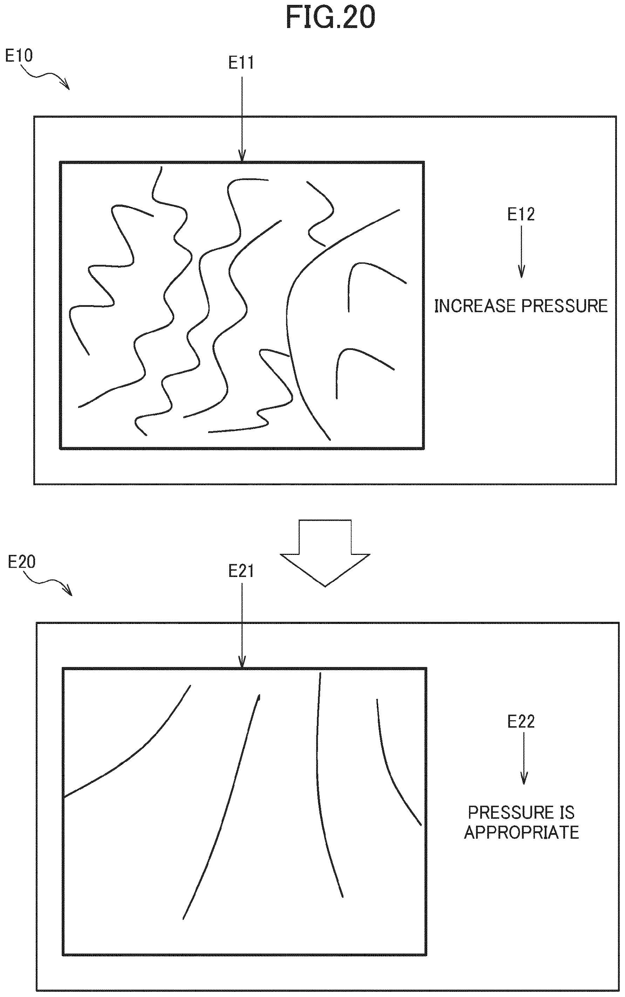

The control section 18 performs control corresponding to a result of determination made by the pressure determination section 32 on the ultrasonic endoscope 100. The pressure determination section 32 will be described later. The control section 18 can be implemented by hardware similar to that of the processor 10. For example, in a case where the endoscope system 1 is motorized, the control section 18 corresponds to a drive control device 200, which will be described later with reference to FIG. 21 or the like. In a case where the endoscope system 1 is not motorized, the control section 18 may also function as a display control section that displays whether or not the pressure is the appropriate pressure on a predetermined display device based on an instruction from the processor 10, and details thereof will be described later with reference to FIG. 20. Note that the control section 18 is arranged separately from the processor 10 in FIG. 17, but may be implemented by hardware identical to that of the processor 10.

The flowchart in FIG. 18 is now described. The endoscope system 1 performs determination processing (step S50). In the determination processing (step S50), specifically, the endoscope system 1 determines whether or not the appropriate pressure is applied to the living body as described in, for example, the flowchart in FIG. 19 (step S52). That is, the endoscope system 1 causes the above-mentioned pressure determination section 32 to function to execute step S52. Therefore, in the endoscope system 1 of the present embodiment, the processor 10 performs the determination processing of determining whether or not the appropriate pressure that guarantees the accuracy of the region marker information is applied to the living body (step S50).

In a case where a result of the determination in the determination processing (step S50) is OK (YES in step S60), the endoscope system 1 performs inference processing (step S70). The inference processing (step S70) is, for example, processing performed by the inference section 30 to output an image in which the detected region marker information is superimposed on the ultrasonic image based on the ultrasonic image as the input data and the trained model 22 as described above with reference to FIG. 10 or the like. That is, in the endoscope system 1 of the present embodiment, when determining that the appropriate pressure is applied to the living body (YES in step S60) in the determination processing (step S50), the processor 10 outputs the region marker information detected based on the ultrasonic image and the trained model, as being superimposed on the ultrasonic image (step S70).

In a case where a result of the determination in the determination processing (step S50) is not OK (NO in step S60), the endoscope system 1 performs pressure optimization processing (step S80). For example, when a manipulation according to the flow in FIG. 7 is started by the ultrasonic endoscope 100, imaging by the imaging sensor through the objective lens 132 described above with reference to FIG. 4 is started. For example, assume that a screen indicated by E10 in FIG. 20 is displayed on the predetermined display device. At this time, the endoscope system 1 causes the pressure determination section 32 to function and determines whether or not the appropriate pressure is applied to the living body based on a captured image indicated by E11. Since the pressure determination section 32 determines that the stomach wall is not sufficiently extended from the captured image indicated by E11, a result of the determination processing (step S50) is not OK.

The endoscope system 1 then performs the pressure optimization processing (step S80). For example, in a case where the endoscope system 1 is not motorized, the endoscope system 1 causes the control section 18 to function to perform display, for example, for prompting the user to increase pressure to the living body as indicated by E12 in FIG. 20 based on an instruction from the processor 10. That is, the control section 18 in this case functions as the display control section that controls the predetermined display device. Although not illustrated, for example, in a case where the endoscope system 1 is motorized, the control section 18 performs, for example, feedback control on the insufflation device, which is not illustrated, to increase a flow rate of the gas. In this manner, in the endoscope system 1 of the present embodiment, the processor 10 performs electric control of the ultrasonic endoscope 100 so as to apply the appropriate pressure.

Thereafter, for example, assume that a screen indicated by E20 in FIG. 20 is displayed on the predetermined display device. The pressure determination section 32 determines that the stomach wall is sufficiently extended from a captured image indicated by E21 in the determination processing (step S50) performed again. Accordingly, a result of the determination processing (step S50) becomes OK. In this case, the endoscope system 1 causes the control section 18 to function in a similar manner to that described above to perform display, for example, indicating that the appropriate pressure is applied to the living body as indicated by E22 in FIG. 20. Therefore, in the endoscope system 1 of the present embodiment, the processor 10 performs presentation processing for presenting a result of the determination processing to the user. With this configuration, the user can determine whether or not the appropriate pressure is applied to the living body.

The method according to the present embodiment may be applied to the motorized endoscope system 1. In other words, operations of each section of the ultrasonic endoscope 100, the biopsy needle 410, and the like may be electrically performed in the above-mentioned method. FIG. 21 illustrates a configuration example of the motorized endoscope system 1. The endoscope system 1 is a system for performing observation or treatment on the inside of the body of the subject lying on an operating table T. The endoscope system 1 includes the ultrasonic endoscope 100, the control device 600, the operation device 300, an advance/retreat drive device 800, and a treatment tool advance/retreat drive device 460, and the display device 900. The control device 600 includes the drive control device 200, and a video control device 500.

The ultrasonic endoscope 100 includes, in addition to the above-mentioned insertion portion 110, a coupling element 125, and an extracorporeal flexible portion 145, connectors 201 and 202. The insertion portion 110, the coupling element 125, the extracorporeal flexible portion 145, and the connectors 201 and 202 are connected to one another in this order from the distal end side.

The insertion portion 110 is, similarly to that in FIG. 1 or the like, a portion inserted into a lumen of the subject, and is configured to be flexible and have a long and thin shape. The insertion portion 110 illustrated in FIG. 21 includes the curved portion 102, an intracorporeal flexible portion 119 that connects a base end of the curved portion 102 and the coupling element 125 to each other, and the distal end portion 130 arranged at the distal end of the curved portion 102. The internal path 101 is arranged inside the insertion portion 110, the coupling element 125, and the extracorporeal flexible portion 145, and a curved wire 160 passes through the internal path 101 and is connected to the curved portion 102. The curved wire 160 will be described later. The drive control device 200 drives the curved wire 160 via the connector 201 to perform a curving operation of the curved portion 102. The raising base operation wire 136, which has been described above with reference to FIG. 5, passes through the internal path 101 and is connected to the connector 201. That is, the drive control device 200 drives the raising base operation wire 136 to change the inclination angle of the raising base 135.

An image signal line that connects an imaging device included in the distal end portion 130 and the connector 202 to each other passes through the internal path 101, and an image signal is transmitted from the imaging device to the video control device 500 via the image signal line. The imaging device is not illustrated. The video control device 500 displays an in-vivo image generated from the image signal on the display device 900. In addition, the ultrasonic cable 159 described above with reference to FIG. 5 passes through the internal path 101, and an echo signal is transmitted from the probe 150 to the video control device 500 via the ultrasonic cable 159. The video control device 500 functions as the above-mentioned ultrasonic observation device, and displays an ultrasonic image generated based on the echo signal on the display device 900. Note that there may be a plurality of display devices 900, and the in-vivo image and the ultrasonic image may be displayed on the respective display devices 900.

In a case where various sensors including the angle sensor, the position sensor, the pressure sensor, and the like are arranged in the distal end portion 130 as described above with reference to FIG. 5, various signal lines that connect the corresponding sensors and the connector 201 are arranged in the internal path 101, and various detection signals are transmitted from the various sensors to the drive control device 200 via the various signal lines.

The insertion opening 190 and a roll operation portion 121 are arranged in the coupling element 125. The roll operation portion 121 is attached to the coupling element 125 to be rotatable about an axis line direction of the insertion portion 110. The rotation operation of the roll operation portion 121 causes roll rotation of the insertion portion 110. As described later, the roll operation portion 121 can be electrically driven.

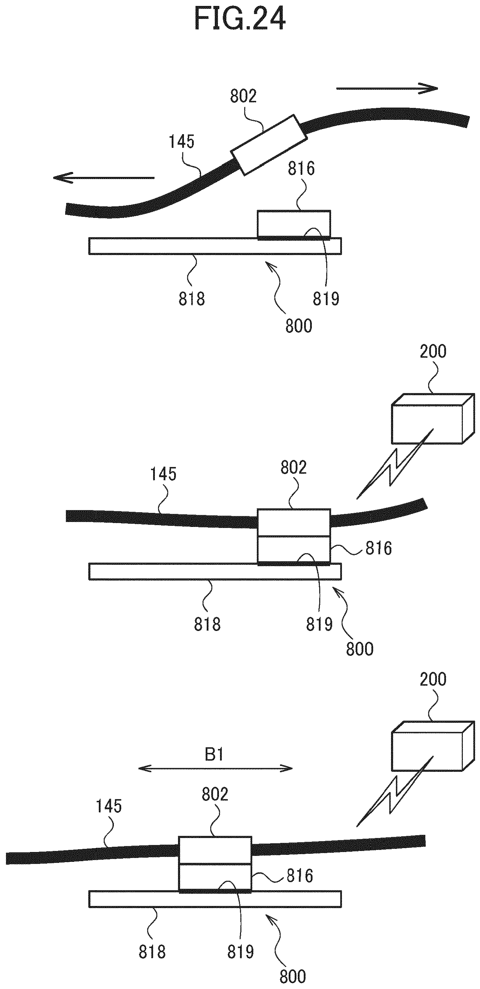

The advance/retreat drive device 800 is a drive device that electrically drives the insertion portion 110 to advance/retreat the insertion portion 110, which will be described later in detail with reference to FIG. 24. The extracorporeal flexible portion 145 is attachable/detachable to/from the advance/retreat drive device 800, and the advance/retreat drive device 800 slides the extracorporeal flexible portion 145 in the axis line direction in a state where the extracorporeal flexible portion 145 is mounted on the advance/retreat drive device 800, whereby the insertion portion 110 advances/retreats. FIG. 24, which will be described later, illustrates an example in which the extracorporeal flexible portion 145 and the advance/retreat drive device 800 are attachable/detachable, but the configuration is not limited thereto, and the coupling element 125 and the advance/retreat drive device 800 may be configured to be attachable/detachable.

The treatment tool advance/retreat drive device 460 is a drive device that electrically drives the treatment tool 400 such as the biopsy needle 410 to advance/retreat, and has, for example, a configuration similar to that of the above-mentioned advance/retreat drive device 800. That is, for example, the sheath portion 411 of the biopsy needle 410 is attachable/detachable to/from the treatment tool advance/retreat drive device 460, and the treatment tool advance/retreat drive device 460 slides the sheath portion 411 in the axis line direction in a state where the sheath portion 411 is mounted on the treatment tool advance/retreat drive device 460, whereby the sheath portion 411 advances/retreats.

The operation device 300 is detachably connected to the drive control device 200 via an operation cable 301. The operation device 300 may perform wireless communication with the drive control device 200 instead of wired communication. When the user operates the operation device 300, a signal of the operation input is transmitted to the drive control device 200 via the operation cable 301, and the drive control device 200 electrically drives the ultrasonic endoscope 100 so as to perform an operation according to the operation input based on the signal of the operation input. The operation device 300 includes operation input sections that correspond to advance/retreat of the ultrasonic endoscope 100, a curving operation and roll rotation in two directions, an operation of the raising base 135, and the like. In a case where there is a non-motorized operation among these operations, an operation input section for the operation may be omitted.

The drive control device 200 drives an actuator such as a built-in motor based on an operation input to the operation device 300 to electrically drive the ultrasonic endoscope 100. Alternatively, in a case where the actuator is an external actuator outside the drive control device 200, the drive control device 200 transmits a control signal to the external actuator based on the operation input to the operation device 300 and controls electric driving. In addition, the drive control device 200 may drive a built-in pump or the like based on the operation input to the operation device 300 and cause the ultrasonic endoscope 100 to perform air supply/aspiration. The air supply/aspiration is performed via an air supply/aspiration tube that passes through the internal path 101. One end of the air supply/aspiration tube opens at the distal end portion 130 of the ultrasonic endoscope 100, and the other end thereof is connected to the drive control device 200 via the connector 201.

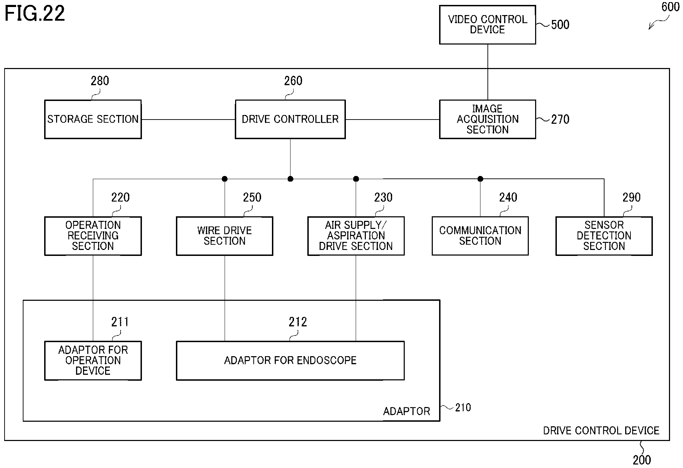

FIG. 22 illustrates a detailed configuration example of the drive control device 200. The drive control device 200 includes an adaptor 210, an operation receiving section 220, an air supply/aspiration drive section 230, a communication section 240, a wire drive section 250, a drive controller 260, an image acquisition section 270, a storage section 280, and a sensor detection section 290.

The adaptor 210 includes an adaptor for the operation device 211 to which the operation cable 301 is detachably connected and an adaptor for the endoscope 212 to which the connector 201 of the ultrasonic endoscope 100 is detachably connected.

The wire drive section 250 performs driving for the curving operation of the curved portion 102 of the ultrasonic endoscope 100 or the operation of the raising base 135, based on a control signal from the drive controller 260. The wire drive section 250 includes a motor unit for the curving operation to drive the curved portion 102 of the ultrasonic endoscope 100 and a motor unit for the raising base to drive the raising base 135. The adaptor for the endoscope 212 has a coupling mechanism for the curving operation for coupling to the curved wire on the ultrasonic endoscope 100 side. The motor unit for the curving operation drives the coupling mechanism, whereby driving force of the driving is transmitted to the curved wire on the ultrasonic endoscope 100 side. The adaptor for the endoscope 212 has a coupling mechanism for the raising base for coupling to the raising base operation wire 136 on the ultrasonic endoscope 100 side. The motor unit for the raising base drives the coupling mechanism, whereby driving force of the driving is transmitted to the raising base operation wire 136 on the ultrasonic endoscope 100 side.

The air supply/aspiration drive section 230 performs driving for air supply/aspiration of the ultrasonic endoscope 100 based on a control signal from the drive controller 260. The air supply/aspiration drive section 230 is connected to the air supply/aspiration tube of the ultrasonic endoscope 100 via the adaptor for the endoscope 212. The air supply/aspiration drive section 230 includes the insufflation device or the like, supplies the air to the air supply/aspiration tube, and aspirates the air from the air supply/aspiration tube.

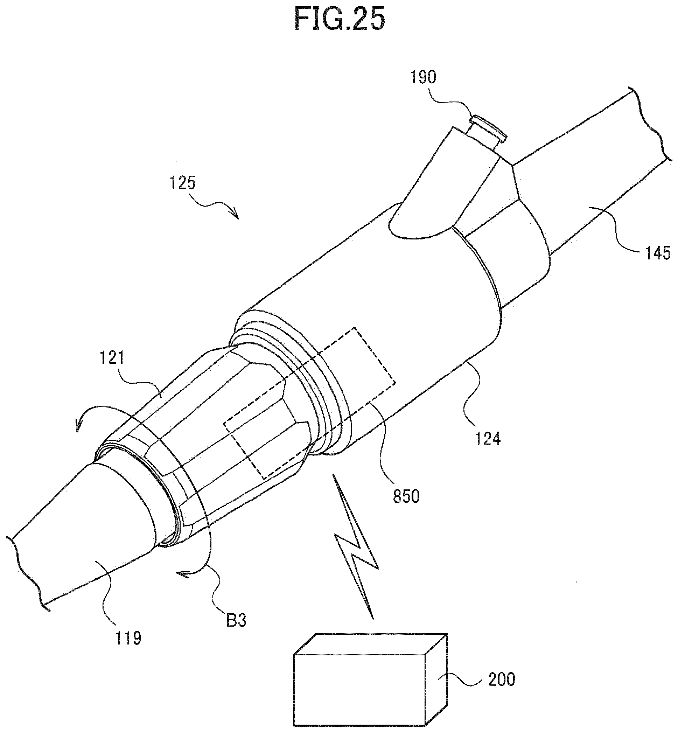

The communication section 240 performs communication with a drive device arranged outside the drive control device 200. Communication may be either wireless communication or wired communication. The external drive device is the advance/retreat drive device 800 that performs advance/retreat, a roll drive device 850 that performs roll rotation, or the like. The roll drive device 850 will be described later with reference to FIG. 25.

The drive controller 260 controls the advance/retreat of the ultrasonic endoscope 100, the curving operation and the roll rotation, the inclination angle of the biopsy needle 410 formed by the raising base 135, and the air supply/aspiration by the ultrasonic endoscope 100. The drive controller 260 is hardware corresponding to the processor 10 illustrated in FIG. 1 or the like.

Additionally, the drive controller 260 controls electric driving based on a signal of an operation input from the operation receiving section 220. Specifically, when the curving operation of the curved portion 102 is performed, the drive controller 260 outputs a control signal indicating a curving direction or a curving angle to the wire drive section 250, and the wire drive section 250 drives the curved wire 160 so that the curved portion 102 is curved in the curving direction or at the curving angle. When advance/retreat is performed, the drive controller 260 transmits a control signal indicating an advance/retreat direction or an advance/retreat movement amount to the advance/retreat drive device 800 via the communication section 240, and the advance/retreat drive device 800 advances/retreats the extracorporeal flexible portion 145 so that the ultrasonic endoscope 100 advances/retreats in the advance/retreat direction or the advance/retreat movement amount. When the roll rotation operation is performed, the drive controller 260 transmits a control signal indicating a roll rotation direction or a roll rotation angle to the roll drive device 850, which will be described later, via the communication section 240, and the roll drive device 850 roll rotates the insertion portion 110 in the roll rotation direction or at the roll rotation angle. Similar control is performed for another electric driving.

The sensor detection section 290 detects a signal for determination about whether the pressure is the above-mentioned appropriate pressure from, for example, an output signal from the above-mentioned various sensors such as the angle sensor, the position sensor, and the pressure sensor. The sensor detection section 290 includes, for example, an amplification circuit that amplifies output signals from the various sensors or the like, and an analog/digital (A/D) converter that performs A/D conversion on an output signal from the amplification circuit and outputs detection data to the drive controller 260. The drive controller 260 performs, for example, control of the inclination angle of the raising base 135 described above with reference to FIG. 5, based on the detection data.

In addition, the drive controller 260 controls the above-mentioned biopsy needle 410 based on the ultrasonic image acquired from the image acquisition section 270 and the signal of the operation input from the operation receiving section 220. In a case of using the above-mentioned machine learning, the above-mentioned trained model 22 is stored in the storage section 280. That is, the storage section 280 in FIG. 22 corresponds to the memory 12 in FIG. 2 or the like.

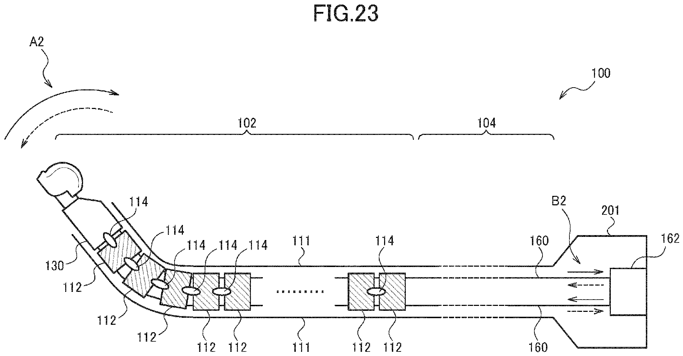

FIG. 23 is a diagram schematically illustrating the ultrasonic endoscope 100 including the curved portion 102 and a drive mechanism for the curved portion 102. The ultrasonic endoscope 100 includes the curved portion 102, the flexible portion 104, and the connector 201, which have been described above. Note that the flexible portion 104 corresponds to the intracorporeal flexible portion 119 and the extracorporeal flexible portion 145, which have been described above, and the coupling element 125 is not illustrated in FIG. 23.

The curved portion 102 and the flexible portion 104 are covered with an outer sheath 111. The inside of the tube of the outer sheath 111 corresponds to the internal path 101 in FIG. 21. The curved portion 102 includes a plurality of curving pieces 112 and the distal end portion 130 that is coupled to a distal end of the curving pieces 112. The plurality of curving pieces 112 and the distal end portion 130 are connected by corresponding pivotable coupling elements 114 in series from the base end side to the distal end side, and have a multiple joint structure. A coupling mechanism 162 on the endoscope side is arranged in the connector 201. The coupling mechanism 162 is connected to a coupling mechanism on the drive control device 200 side. The connector 201 is mounted on the drive control device 200, whereby it becomes possible to perform electric driving for the curving operation. The curved wire 160 is arranged inside the outer sheath 111. One end of the curved wire 160 is connected to the distal end portion 130. The curved wire 160 penetrates the plurality of curving pieces 112, passes the flexible portion 104, folds back inside the coupling mechanism 162, passes the flexible portion 104 again, and penetrates the plurality of curving pieces 112. The other end of the curved wire 160 is connected to the distal end portion 130. Driving force from the wire drive section 250 is transmitted as tractive force of the curved wire 160 to the curved wire 160 via the coupling mechanism 162.

As indicated by an arrow of a solid line in B2, when a wire on an upper side of the drawing is pulled, a wire on a lower side of the drawing is pushed, whereby a multiple joint of the curving pieces 112 is bent in an upper direction of the drawing. With this operation, as indicated by an arrow of a solid line in A2, the curved portion 102 is curved in the upper direction of the drawing. In a case where the wire on the lower side of the drawing is pulled as indicated by an arrow of a dotted line in B2, the curved portion 102 is similarly curved in a lower direction of the drawing as indicated by a dotted line in A2. Note that the curved portion 102 is capable of being curved independently in two directions that are orthogonal to each other. FIG. 23 illustrates a curving mechanism only for one direction, but two sets of curved wires are actually arranged. Each curved wire is pulled independently by the coupling mechanism 162, and can thereby be curved independently in two directions.

Note that a mechanism for electrical driving for curving is not limited to the above-mentioned mechanism. For example, a motor unit may be arranged in substitution for the coupling mechanism 162. Specifically, the drive control device 200 transmits a control signal to the motor unit via the connector 201 and the motor unit may perform driving for the curving operation by pulling or loosening the curved wire 160 based on the control signal.

FIG. 24 illustrates a configuration example of the advance/retreat drive device 800. The advance/retreat drive device 800 includes a motor unit 816, a base 818, and a slider 819.

As illustrated in an upper drawing and a middle drawing, the extracorporeal flexible portion 145 of the ultrasonic endoscope 100 is provided with an attachment 802 that is detachably mounted on the motor unit 816. As illustrated in the middle drawing, the attachment 802 is mounted on the motor unit 816, whereby it becomes possible to perform electric driving for advance/retreat. As illustrated in a lower drawing, the slider 819 supports the motor unit 816 so as to be linearly movable with respect to the base 818. The slider 819 is fixed to the operating table T illustrated in FIG. 21. As indicated by B1, the drive control device 200 transmits a control signal for advance/retreat to the motor unit 816 through wireless communication, and the motor unit 816 and the attachment 802 linearly move over the slider 819 based on the control signal. With this configuration, advance/retreat of the insertion portion 110 can be implemented. Note that the drive control device 200 and the motor unit 816 may have a wired connection.

Although not illustrated, the treatment tool advance/retreat drive device 460 may also be configured to include a motor unit, a base, and a slider in a similar manner. In addition, an attachment detachably mounted on the motor unit may be arranged in the sheath portion 411 of the biopsy needle 410. Although not illustrated, each of the needle and stylet of the needle portion 412 included in the biopsy needle 410 may be electrically controlled. For example, each of the needle and the stylet described above is connected to a motorized cylinder. The drive control device 200 then transmits a predetermined control signal to the motorized cylinder, and the needle and the stylet operate based on the control signal. Either the needle or the stylet may be electrically controlled.

For example, the method described with reference to FIG. 8 may be combined with the ultrasonic endoscope 100 and the biopsy needle 410 that are electrically controlled in this manner. With this configuration, for example, the needle portion 412 of the biopsy needle 410 can be electrically inserted toward the position of the lesion corresponding to the position indicated by C21 in the ultrasonic image indicated by C20 in FIG. 8. That is, in the endoscope system 1 of the present embodiment, the processor 10 performs electrical control of inserting the biopsy needle 410 into the lesion based on the calculated angle of the biopsy needle 410. With this control, it becomes possible to construct a system of electrically inserting the biopsy needle 410 into the lesion while directing the biopsy needle 410 at an optimal angle with respect to the lesion.

Similarly, the method described with reference to FIG. 9 may be combined with the ultrasonic endoscope 100 and the biopsy needle 410 that are electrically controlled. That is, in the endoscope system 1 of the present embodiment, the processor 10 performs control of electrically inserting the biopsy needle 410 into the lesion based on the calculated angle and depth of the biopsy needle 410. With this control, it becomes possible to construct a system of electrically inserting the biopsy needle 410 into the lesion in an appropriate stroke amount while directing the biopsy needle 410 at an optimal angle with respect to the lesion.

FIG. 25 is a perspective view illustrating the coupling element 125 including the roll drive device 850. The coupling element 125 includes a coupling element main body 124 and the roll drive device 850.

The insertion opening 190 is arranged in the coupling element main body 124, and is connected to the treatment tool insertion path, which is not illustrated in FIG. 25, inside the coupling element main body 124. The coupling element main body 124 has a cylindrical shape, and a cylindrical member that is coaxial with a cylinder of the coupling element main body 124 is rotatably arranged inside the coupling element main body 124. A base end portion of the intracorporeal flexible portion 119 is fixed to the outside of the cylindrical member, and the base end portion serves as the roll operation portion 121. This allows the intracorporeal flexible portion 119 and the cylindrical member are rotatable with respect to the coupling element main body 124 about the axis line direction of the intracorporeal flexible portion 119. The roll drive device 850 is the motor unit arranged inside the coupling element main body 124. As indicated by B3, the drive control device 200 transmits a control signal for roll rotation to the roll drive device 850 through wireless communication, and the roll drive device 850 rotates the base end portion of the intracorporeal flexible portion 119 with respect to the coupling element main body 124 based on the control signal, whereby the intracorporeal flexible portion 119 roll rotates. Note that the roll drive device 850 may include a clutch mechanism, which switches between non-electric driving and electric driving of the roll rotation. Note that the drive control device 200 and the roll drive device 850 may have a wired connection using a signal line that passes through the internal path 101.

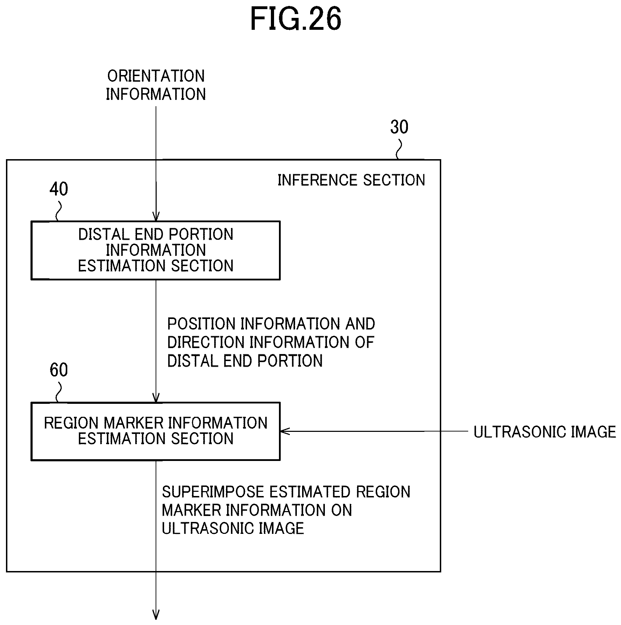

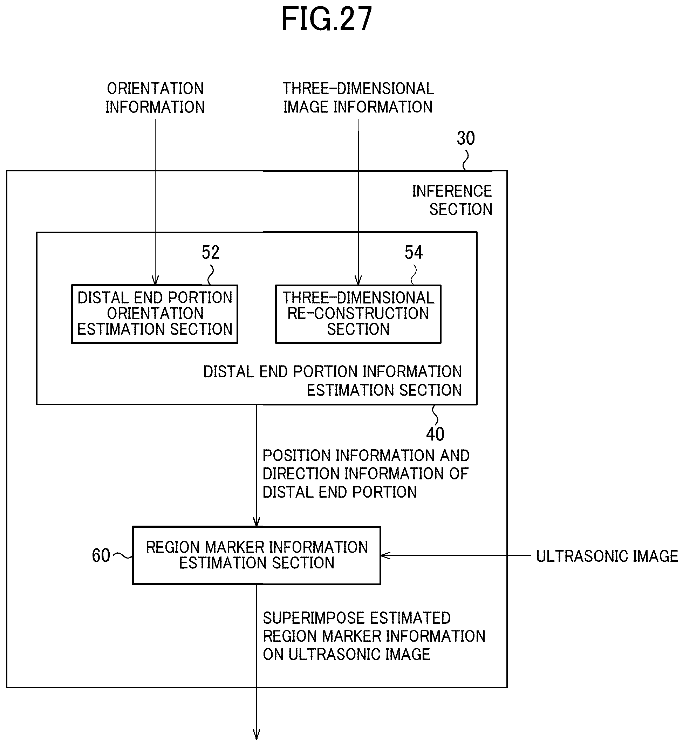

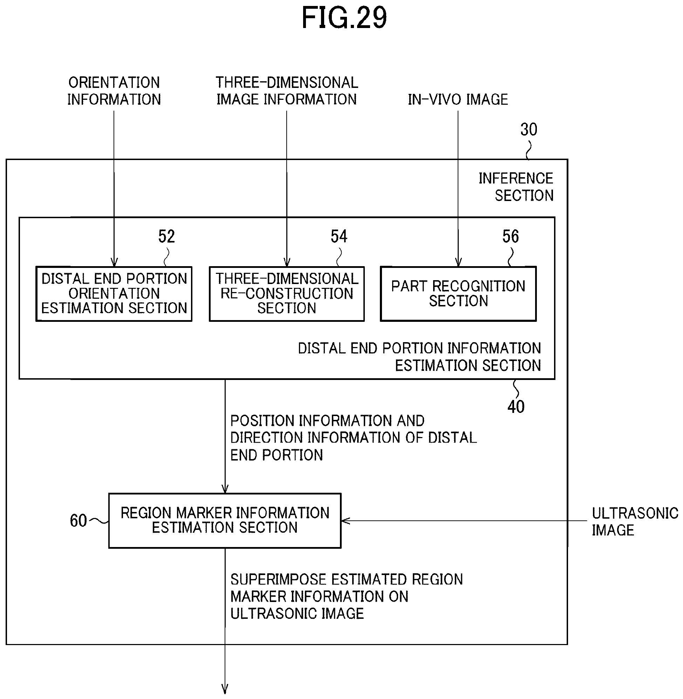

The method according to the present embodiment is not limited thereto, and the region marker information may be detected, for example, using the ultrasonic image and another data as the input data. More specifically, for example, the inference section 30 described with reference to FIG. 10 or the like may have a configuration in a configuration example illustrated in FIG. 26. The inference section 30 includes a distal end portion information estimation section 40 and a region marker information estimation section 60. The description with reference to FIG. 26 is on the assumption that the main section that performs processing is each section illustrated in FIG. 26, but the main section that performs processing can be replaced by the endoscope system 1 or the processor 10 as appropriate. The same applies to FIG. 27, FIG. 29, and the like.

The distal end portion information estimation section 40 receives orientation information of the distal end portion 130, and transmits position information and direction information of the distal end portion 130 acquired based on the orientation information to the region marker information estimation section 60. The orientation information of the distal end portion 130 mentioned herein is, for example, measurement data obtained by an inertial measurement unit (IMU) arranged at a predetermined position of the distal end portion 130. The IMU is an inertial sensor unit including a speed sensor and a gyro sensor. The speed sensor and the gyro sensor can be implemented by, for example, a micro electro mechanical systems (MEMS) sensor or the like. The distal end portion information estimation section 40 acquires six degrees of freedom (6DoF) information as the position information and direction information of the distal end portion 130 based on, for example, the measurement data from the IMU. For example, a magnetic field that occurs from a coil arranged in a predetermined relationship with the insertion portion 110 or the like including the distal end portion 130 is detected from an antenna, which is not illustrated, and information based on a detection signal from the antenna may serve as the orientation information of the distal end portion 130. For example, the distal end portion information estimation section 40 functions as a UPD device, and acquires the orientation information of the insertion portion 110 or the like including the distal end portion 130 as the position information and direction information of the insertion portion 110 or the like from amplitude, a phase, or the like of the detection signal. The UPD device is also referred to as an endoscope position detecting unit.

The region marker information estimation section 60 reads out the trained model 22 from the memory 12, performs the inference processing (step S70) in FIG. 18 using the ultrasonic image and the position information and direction information of the distal end portion 130 as the input data to detect the region marker information, and outputs the ultrasonic image on which the detected region marker information is superimposed. That is, the position information and direction information of the distal end portion 130 is metadata to increase accuracy of inference. In this manner, in the endoscope system of the present embodiment, the processor 10 detects the region marker information based on the position information and direction information of the probe 150 of the ultrasonic endoscope 100, the ultrasonic image, and the trained model 22. With this configuration, it becomes possible to construct the endoscope system 1 that detects the region marker information based on the position information and direction information of the probe 150. This can increase the accuracy of detection of the region marker information.

Since the ultrasonic endoscope 100 is used in the present embodiment, the orientation information or the like of the distal end portion 130 described above can also be replaced by the orientation information or the like of the probe 150. The same applies to the subsequent description.

These pieces of input data may be used in a training phase. That is, in a case where the inference processing (step S70) is performed by the inference section 30 in FIG. 26, the training data 84 in the training device 3 in FIG. 15 is the position information and direction information of the probe 150 and the ultrasonic image.

For example, the inference section 30 may have a configuration in a configuration example illustrated in FIG. 27. The distal end portion information estimation section 40 in FIG. 27 is different from the configuration example in FIG. 25 in that the distal end portion information estimation section 40 includes a distal end portion orientation estimation section 52 and a three-dimensional re-construction section 54. Note that in FIG. 27, a description about a configuration, processing, and the like that overlap with those in FIG. 26 is omitted as appropriate. The distal end portion orientation estimation section 52 in FIG. 27 uses the IMU or the like described with reference to FIG. 26 acquires the position information and direction information of the distal end portion 130 based on the orientation information of the distal end portion 130.

The three-dimensional re-construction section 54 acquires three-dimensional re-construction data based on three-dimensional image information. The three-dimensional image information mentioned herein is image information in which the position of each pixel is defined by a three-dimensional coordinate system, and is, for example, image information captured and acquired by, for example, a method of computed tomography (CT), magnetic resonance imaging (MRI), positron emission tomography (PET), or the like. The three-dimensional image information is preliminarily acquired by the above-mentioned method performed on the living body as the subject. The three-dimensional image information may be stored in, for example, the memory 12 or may be stored in, for example, a database of an external device, or the like. The three-dimensional re-construction section 54 re-constructs the three-dimensional shape information of the living body from the three-dimensional image information by a method of volume rendering or the like.

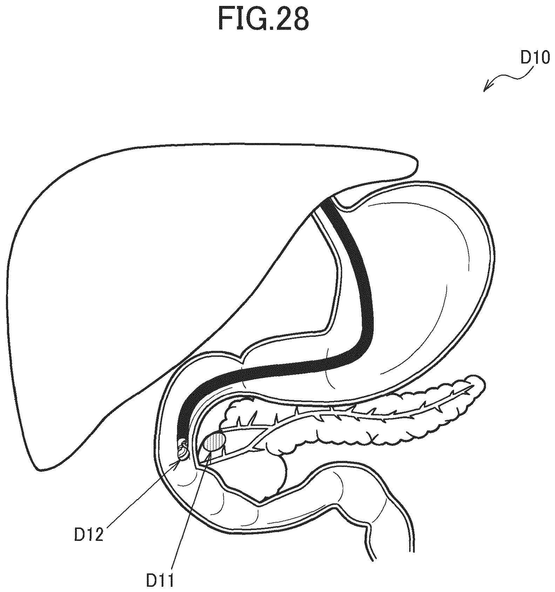

Note that the position information and direction information of the distal end portion 130 integrated by the distal end portion orientation estimation section 52 and the three-dimensional re-construction section 54 may be used to construct, for example, a model as indicated by D10 in FIG. 28. Alternatively, the model indicated by D10 in FIG. 28 may be displayed next to the ultrasonic image on the predetermined display device. With this configuration, given that the user performs, for example, an ultrasonic diagnosis on a location including an object indicated by D11 in FIG. 28, he/she can confirm that the distal end portion 130 is located at a position indicated by D12, that the probe 150 is directed in a direction toward the object indicated by D11 or the like, and other matters. This allows the user to confirm that the displayed ultrasonic image is an image in which a predetermined part is drawn. This can reduce a psychological burden on the user.

In this manner, the distal end portion information estimation section 40 illustrated in FIG. 27 transmits the position information and direction information of the distal end portion 130 based on the orientation information of the distal end portion 130 and the three-dimensional image information to the region marker information estimation section 60. That is, in the endoscope system 1 of the present embodiment, the processor 10 obtains the position information and direction information of the ultrasonic endoscope 100 based on the three-dimensional shape information of the living body and the orientation information of the probe 150 of the ultrasonic endoscope 100. With this configuration, it becomes possible to construct a system of acquiring the position information and direction information of the probe 150 based on the three-dimensional image information of the living body and the orientation information of the probe 150. This can increase the accuracy of detection of the region marker information.

For example, the inference section 30 may have a configuration in a configuration example illustrated in FIG. 29. The distal end portion information estimation section 40 illustrated in FIG. 29 is different from the configuration example in FIG. 27 in that the distal end portion information estimation section 40 further includes a part recognition section 56. Note that in FIG. 29, a description about a configuration, processing, and the like that overlap with those in FIGS. 26 and 27 is omitted as appropriate.

The part recognition section 56 illustrated in FIG. 29 acquires information regarding the position of the distal end portion 130 and the direction in which the distal end portion 130 is directed based on the in-vivo image. The in-vivo image is an image that is captured by the imaging device included in the distal end portion 130 and in which the inside of a lumen in the living body as the subject is imaged. Since a texture of the inner wall of the lumen is different depending on the part whose image is captured, the in-vivo image provides the information regarding the position and direction of the distal end portion 130. In this manner, the distal end portion information estimation section 40 illustrated in FIG. 29 acquires the position information and direction information of the distal end portion 130 based on the orientation information of the distal end portion 130, the three-dimensional image information, and the information regarding the living body, and transmits these pieces of information to the region marker information estimation section 60. Therefore, in the endoscope system 1 of the present embodiment, the processor 10 obtains the position information and direction information of the ultrasonic endoscope 100 based on the three-dimensional shape information of the living body, the orientation information of the probe 150 of the ultrasonic endoscope 100, and an in-vivo image captured by the imaging sensor of the ultrasonic endoscope 100. With this configuration, it becomes possible to construct a system of obtaining the position information and direction information of the probe 150 based on the orientation information of the probe 150, the three-dimensional shape information of the living body, and the in-vivo image. The region marker information estimation section 60 then uses the acquired position information and direction information of the distal end portion 130 and the ultrasonic image as an input data set, detects the region marker information by a method similar to that described with reference to FIGS. 25 and 26, and outputs the ultrasonic image on which the detected region marker information is superimposed.

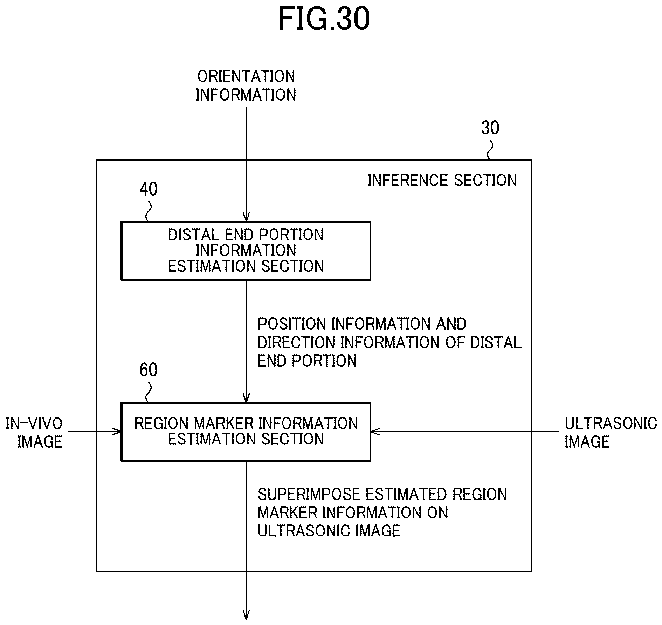

The inference section 30 of the present embodiment may have a configuration in a configuration example illustrated in FIG. 30. The configuration example illustrated in FIG. 30 is different from the configuration example illustrated in FIG. 26 in that it has a feature that the in-vivo image described above with reference to FIG. 29 serves as metadata to be further input to the region marker information estimation section 60. Note that the feature may be added to the configuration example illustrated in FIG. 27, or may be added to the configuration example illustrated in FIG. 29. In this manner, in the endoscope system 1 of the present embodiment, the processor 10 detects the region marker information based on the position information and direction information of the probe 150 of the ultrasonic endoscope 100, the ultrasonic image, the in-vivo image captured by the imaging sensor of the ultrasonic endoscope 100, and the trained model 22. With this configuration, it becomes possible to construct the endoscope system 1 that detects the region marker information based on the position information and direction information of the probe 150, the ultrasonic image, and the in-vivo image. This can increase the accuracy of detection of the region marker information.

These pieces of input data may be used in a training phase. That is, in a case where the inference processing (step S70) is performed by the inference section 30 in FIG. 30, the training data 84 in the training device 3 in FIG. 15 is the position information and direction information of the probe 150, the ultrasonic image, and the in-vivo image.

Although illustration or the like of a configuration example of the inference section 30 is omitted, the endoscope system 1 of the present embodiment may use, as the input data set, the ultrasonic image and the in-vivo image as metadata to perform the inference processing (step S70) or the like. In this case, the training data 84 in the training phase is the ultrasonic image and the in-vivo image. That is, in the endoscope system 1 of the present embodiment, the trained model 22, to which the ultrasonic image and the in-vivo image captured by the imaging sensor of the ultrasonic endoscope 100 are input, is trained to output the region marker information. With this configuration, it becomes possible to construct the trained model 22 that detects the region marker information based on the ultrasonic image and the in-vivo image. This can increase the accuracy of detection of the region marker information.

Additionally, the endoscope system 1 of the present embodiment may be capable of presenting operation support information to insert the biopsy needle 410. That is, in the endoscope system 1 of the present embodiment, the processor 10 performs presentation processing for presenting the operation support information for the ultrasonic endoscope 100 to the user based on the calculated angle and depth of the biopsy needle 410. This can reduce a work burden on the user to insert the biopsy needle 410 into the lesion.

FIG. 31 is a flowchart describing a processing example of presentation processing for presenting operation support information for the ultrasonic endoscope 100. The endoscope system 1 determines whether or not the lesion and the important tissue can be detected (step S10). In a case where the lesion and the important tissue can be detected (YES in step S10), the endoscope system 1 performs processing in step S20 or subsequent steps. In a case where the lesion and the important tissue cannot be detected (NO in step S10), the endoscope system 1 performs step S10 again. Specifically, for example, the endoscope system 1 determines whether or not the region marker information is superimposed on the ultrasonic image, and determines that a result is YES in step S10 at timing when the region marker information is superimposed on the ultrasonic image.

After determining that the result is YES in step S10, the endoscope system 1 compares the positions of the lesion and important tissue and the movable range of the biopsy needle 410 (step S20). If determining that the lesion does not exist in the movable range (NO in step S30), the endoscope system 1 performs first notification (step S110), and ends the flow. In contrast, if determining that the lesion exists in the movable range (YES in step S30), the endoscope system 1 determines whether or not the important tissue exists in the movable range (step S40). If determining that the important tissue exists in the movable range (YES in step S40), the endoscope system 1 performs second notification (step S120), and ends the flow. In contrast, if determining that the important tissue does not exist in the movable range (NO in step S40), the endoscope system 1 performs third notification (step S130), and ends the flow.

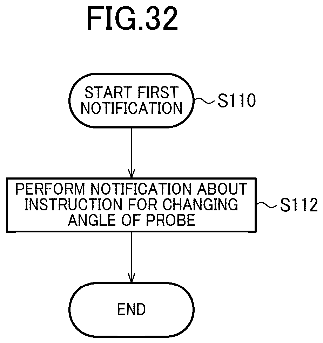

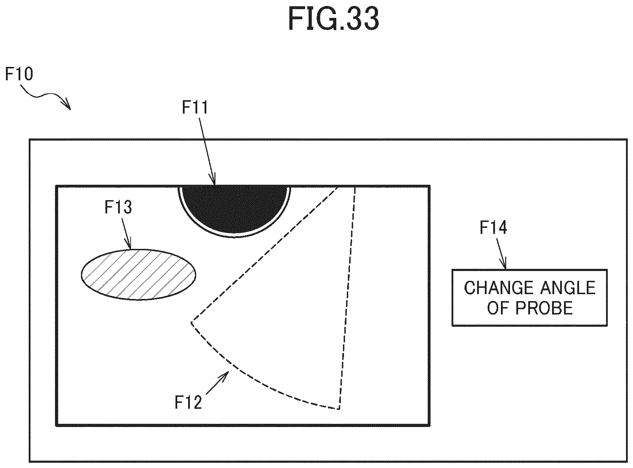

The first notification (step S110) is, specifically, to notify an instruction for changing the angle of the probe 150 as described in a flowchart in FIG. 32 (step S112). For example, assume that an ultrasonic image indicated by F11 is displayed on a screen indicated by F10 in FIG. 33. A movable range image indicated by F12 and region marker information indicated by F13 are displayed in the ultrasonic image indicated by F11. Assume that the region marker information indicated by F13 is region marker information corresponding to the lesion.

Since the movable range image indicated by F12 is not superimposed on the region marker information indicated by F13, the endoscope system 1 executes step S112. With this processing, for example, a message indicated by F14 is displayed on the screen indicated by F10. In this case, for example, the user performs an operation of curving the curved portion 102 in an upper direction on the paper, whereby the movable range image indicated by F12 is superimposed on the region marker information of the lesion indicated by F13. In this manner, in the endoscope system 1 of the present embodiment, the processor 10 determines whether or not the lesion is included in the movable range of the biopsy needle 410. In a case where the lesion is not included in the movable range, the processor 10 outputs instruction information to change the angle of the probe 150 of the ultrasonic endoscope 100. With this configuration, in a case where the lesion is not included in the movable range of the biopsy needle 410, the user can recognize that he/she can perform appropriate handling by changing the angle of the probe 150.

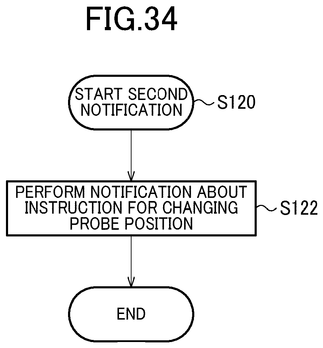

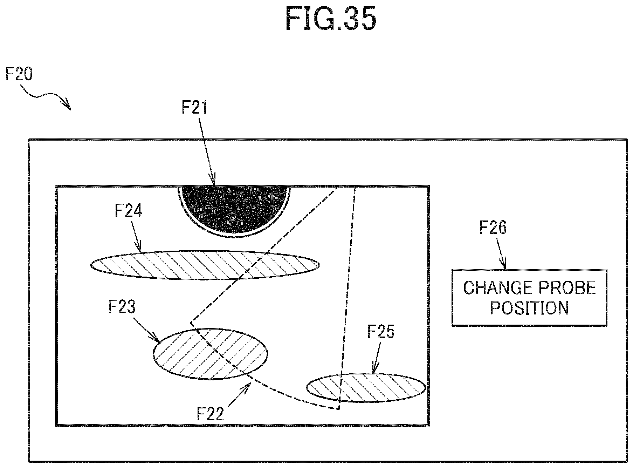

The second notification (step S120) is, specifically, to notify an instruction for changing the position of the probe 150 as described in a flowchart in FIG. 34 (step S122). For example, assume that an ultrasonic image indicated by F21 is displayed on a screen indicated by F20 in FIG. 35. A movable range image indicated by F22, region marker information indicated by F23, region marker information indicated by F24, and region marker information indicated by F25 are displayed in the ultrasonic image indicated by F21. Assume that the region marker information indicated by F23 mentioned herein is the region marker information corresponding to the lesion, and each of the region marker information indicated by F24 and the region marker information indicated by F25 is region marker information corresponding to the important tissue.

Since the movable range image indicated by F22 is superimposed on the region marker information indicated by F23, it means a situation in which the biopsy needle 410 can be inserted into the lesion by being projected. However, the movable range image indicated by F22 is also superimposed on the region marker information indicated by F24 and the region marker information indicated by F25. The region marker information indicated by F24 can be located between the projection position of the biopsy needle 410 and the region marker information indicated by F23. If the biopsy needle 410 is projected under such a situation, the biopsy needle 410 is inserted into the important tissue, and there is a possibility that the important tissue is damaged.

Under such a situation, the endoscope system 1 executes step S122. With this processing, for example, a message indicated by F26 is displayed on the screen indicated by F20. Under a situation in FIG. 35, unlike the situation in FIG. 33, there is a case where it is impossible to superimpose only the region marker information corresponding to the lesion on the movable range image even if the angle of the probe 150 is changed. To address this, the processor 10 performs notification indicated by F26 to prompt the user to retreat the insertion portion 110 once and change an approach method with respect to the lesion. Therefore, in the endoscope system 1 of the present embodiment, the processor 10 determines whether or not the important tissue is included between the projection position of the biopsy needle 410 and the lesion. In a case where the important tissue is included between the projection position of the biopsy needle 410 and the lesion, the processor 10 outputs instruction information to change the position of the ultrasonic endoscope 100. With this configuration, in a case where the important tissue is included between the projection position of the biopsy needle 410 and the lesion, the user can recognize that it is in a difficult situation to project the biopsy needle 410 unless the approach method for causing the probe 150 to approach the lesion is changed. This allows the user to more appropriately perform the treatment using the biopsy needle 410.

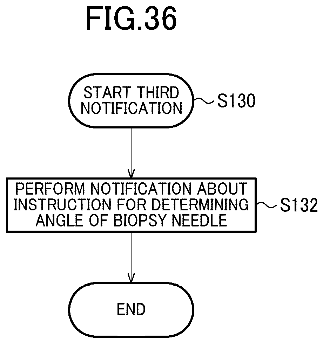

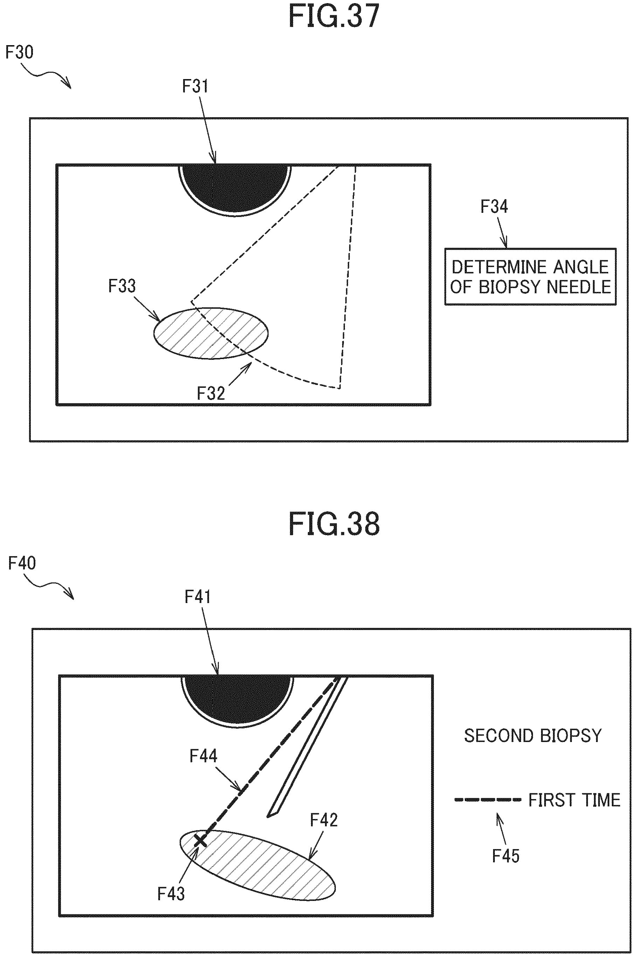

The third notification (step S130) is, specifically, to make notification to the user to prompt the user to determine the insertion angle of the biopsy needle 410 as described in a flowchart in FIG. 36 (step S132). For example, assume that an ultrasonic image indicated by F31 is displayed on a screen indicated by F30 in FIG. 37. A movable range image indicated by F32 and region marker information indicated by F33 are displayed in the ultrasonic image indicated by F31. Assume that the region marker information indicated by F33 is the region marker information corresponding to the lesion.

Since the movable range image indicated by F32 is superimposed on the region marker information indicated by F33, it means a situation in which the biopsy needle 410 can be inserted into the lesion by being projected. Under a situation illustrated in FIG. 37, unlike the situation illustrated in FIG. 35, the movable range image indicated by F32 and the region marker information corresponding to the important tissue are not superimposed on each other. Thus, it is in a situation where the biopsy needle 410 can be inserted into the lesion by being projected without causing a damage on the important tissue. Under such a situation, the endoscope system 1 executes step S132. With this configuration, for example, a message that the angle of the biopsy needle 410 needs to be determined as indicated by F34 is displayed on the screen indicated by F30. Thereafter, the user determines the angle of the biopsy needle 410 by the method or the like described with reference to FIG. 8, FIG. 9, and the like. That is, in the endoscope system 1 of the present embodiment, the processor 10 determines whether or not the lesion and the important tissue are included in the movable range of the biopsy needle 410. In a case where the lesion is included in the movable range and the important tissue is not included in the movable range, the processor 10 performs control of inserting the biopsy needle 410 into the lesion. This allows the user to recognize that it is in a situation where the biopsy needle 410 can be projected without a problem.

The endoscope system 1 of the present embodiment may display another operation support information. The other operation support information is, for example, operation support information to insert the biopsy needle 410 multiple times. As a modification of the operation support information, for example, in a case where the biopsy needle 410 is inserted at a predetermined location of the lesion and is inserted again at a different location of the lesion, a screen as indicated by F40 in FIG. 38 may be displayed. An ultrasonic image indicated by F41 and an explanatory note indicated by F45 are displayed in the screen indicated by F40. Region marker information indicated by F42, an icon indicated by F43, and a dotted line icon indicated by F44 are displayed in the ultrasonic image indicated by F41. This indicates that the user has inserted the biopsy needle 410 at a position corresponding to the icon indicated by F43 in the first biopsy (step S5), and the icon indicated by F44 indicates a path through which the biopsy needle 410 has passed. This allows the user to recognize that the angle of the biopsy needle 410 is determined so as to be different from the angle of the biopsy needle 410 based on the dotted line indicated by F44. Note that the icon indicated by F43 can be implemented by, for example, processing of creating the icon based on coordinates of the specific position described above with reference to FIG. 8. Similarly, the icon indicated by F44 can be implemented by processing of creating the icon based on the second straight line described above with reference to FIG. 8.

Although not illustrated, for example, the endoscope system 1 may determine whether or not the biopsy needle 410 has been inserted into the lesion. For example, the endoscope system 1 performs processing of detecting, in addition to the region marker information corresponding to the lesion, region marker information corresponding to the biopsy needle 410. The endoscope system 1 then performs processing of determining whether or not the region marker information corresponding to the biopsy needle 410 and the region marker information corresponding to the lesion are superimposed on each other, and can thereby determine whether or not the biopsy needle 410 has been inserted into the lesion by image processing. Instead of the region marker information corresponding to the biopsy needle 410, the endoscope system 1 may create an image of part of the above-mentioned first straight line for a projection length of the needle portion 412 and display the image. Even if the created image of the first straight line is displayed in conjunction with the stroke amount of the needle portion 412, a similar effect can be obtained.

## Claims

1. A processing apparatus comprising:
a processor (10) including hardware, the processor (10) being configured to:
acquire an image in which region marker information of a lesion is set with respect to an ultrasonic image of an ultrasonic endoscope (100) with a biopsy needle (410);
the processor (10) being configured to:
calculate an angle of the biopsy needle (410) for inserting the biopsy needle (410) into the lesion based on a movable range (R3) of the biopsy needle (410) and the region marker information;
**characterised in that** the processor is further configured to:
determine whether the lesion is included in the movable range (R3); and
where the lesion is not included in the movable range (R3), output instruction information to change an angle of a probe (150) of the ultrasonic endoscope (100); and/or
wherein the processor (10) is further configured to:
determine whether the lesion and a predetermined tissue are included in the movable range (R3); and
where the lesion is included in the movable range (R3) and the predetermined tissue is not included in the movable range (R3), perform control of inserting the biopsy needle (410) into the lesion; and/or
wherein the processor (10) is further configured to:
determine whether a predetermined tissue is included between a projection position of the biopsy needle (410) and the lesion; and
where the predetermined tissue is included between the projection position of the biopsy needle (410) and the lesion, output instruction information to change a probe (150) position of the ultrasonic endoscope (100).

2. The processing apparatus of claim 1, wherein the processor (10) is configured to control an actuator to be directed toward the lesion based on the calculated angle of the biopsy needle (410).

3. The processing apparatus of claim 1 or 2, wherein the processor (10) is configured to perform presentation processing for presenting operation support information of the ultrasonic endoscope (100) to a user based on the calculated angle of the biopsy needle (410).

4. The processing apparatus of anyone of claims 1 to 3, wherein the processor (10) is configured to calculate an angle and depth of the biopsy needle (410) for inserting the biopsy needle (410) into the lesion based on the movable range (R3) of the biopsy needle (410) and the region marker information.

5. The processing apparatus of claim 4, wherein the processor (10) is configured to control an actuator to electrically insert the biopsy needle (410) into the lesion based on the calculated angle and depth of the biopsy needle (410).

6. The processing apparatus of claim 4 or 5, wherein the processor (10) is configured to perform presentation processing for presenting operation support information of the ultrasonic endoscope (100) to a user based on the calculated angle and depth of the biopsy needle (410).

7. The processing apparatus of anyone of claims 1 to 6, wherein the region marker information includes marker information corresponding to a region of the lesion and marker information corresponding to a region of a predetermined tissue.

8. The processing apparatus of anyone of claims 1 to 7, further comprising a memory (12) that stores a trained model (22) trained so as to output, to the ultrasonic image captured by the ultrasonic endoscope (100), the region marker information of a detection target in the ultrasonic image,
wherein the processor (10) is configured to detect the region marker information based on the ultrasonic image and the trained model (22).

9. A computer program configured to be run by a processing apparatus, the method comprising:
acquiring an image in which region marker information of a lesion is set with respect to an ultrasonic image of an ultrasonic endoscope (100) with a biopsy needle (410);
calculating an angle of the biopsy needle (410) for inserting the biopsy needle (410) into the lesion based on a movable range (R3) of the biopsy needle (410) and the region marker information;
**characterised in that** the computer program further comprises
determining whether the lesion is included in the movable range (R3); and where the lesion is not included in the movable range (R3), output instruction information to change an angle of a probe (150) of the ultrasonic endoscope (100); and/or
determining whether the lesion and a predetermined tissue are included in the movable range (R3); and where the lesion is included in the movable range (R3) and the predetermined tissue is not included in the movable range (R3), perform control of inserting the biopsy needle (410) into the lesion; and/or
determining whether a predetermined tissue is included between a projection position of the biopsy needle (410) and the lesion; and where the predetermined tissue is included between the projection position of the biopsy needle (410) and the lesion, output instruction information to change a probe (150) position of the ultrasonic endoscope (100).

10. The computer program of claim 9, comprising:
presenting operation support information of the ultrasonic endoscope (100) to a user based on the calculated angle of the biopsy needle (410); and/or
calculating an angle and depth of the biopsy needle (410) for inserting the biopsy needle (410) into the lesion based on the movable range (R3) of the biopsy needle (410) and the region marker information; and/or
presenting of operation support information of the ultrasonic endoscope (100) to a user based on the calculated angle and depth of the biopsy needle (410).

11. The computer program of anyone of claims 9 to 10, wherein the region marker information includes marker information corresponding to a region of the lesion and marker information corresponding to a region of an important tissue.

12. The computer program of anyone of claims 9 to 11, comprising detecting the region marker information based on a trained model (22) trained so as to output, to the ultrasonic image captured by the ultrasonic endoscope (100), the region marker information of a detection target in the ultrasonic image, and the ultrasonic image.

## Patentansprüche

1. Verarbeitungsvorrichtung, die umfasst:
einen Prozessor (10) mit Hardware, wobei der Prozessor (10) konfiguriert ist:
ein Bild zu erfassen, in dem eine Bereichsmarkierungsinformation einer Läsion in Bezug auf ein Ultraschallbild eines Ultraschallendoskops (100) mit einer Biopsienadel (410) gesetzt ist;
wobei der Prozessor (10) konfiguriert ist:
einen Winkel der Biopsienadel (410) zum Einführen der Biopsienadel (410) in die Läsion auf der Grundlage eines Bewegungsbereichs (R3) der Biopsienadel (410) und der Bereichsmarkierungsinformation zu berechnen;
**dadurch gekennzeichnet, dass** der Prozessor ferner konfiguriert ist:
zu bestimmen, ob die Läsion in dem Bewegungsbereich (R3) enthalten ist; und
wenn die Läsion nicht in dem Bewegungsbereich (R3) enthalten ist, eine Befehlsinformation zum Ändern eines Winkels einer Sonde (150) des Ultraschallendoskops (100) auszugeben; und/oder
wobei der Prozessor (10) ferner konfiguriert ist:
zu bestimmen, ob die Läsion und ein vorbestimmtes Gewebe in dem Bewegungsbereich (R3) enthalten sind; und
wenn die Läsion in dem Bewegungsbereich (R3) enthalten ist und das vorbestimmte Gewebe nicht in dem Bewegungsbereich (R3) enthalten ist, eine Steuerung zum Einführen der Biopsienadel (410) in die Läsion durchzuführen; und/oder
wobei der Prozessor (10) ferner konfiguriert ist:
zu bestimmen, ob ein vorbestimmtes Gewebe zwischen einer Projektionsposition der Biopsienadel (410) und der Läsion enthalten ist; und
wenn das vorbestimmte Gewebe zwischen der Projektionsposition der Biopsienadel (410) und der Läsion enthalten ist, eine Befehlsinformation zum Ändern eine Position einer Sonde (150) des Ultraschallendoskops (100) auzugeben.

2. Verarbeitungsvorrichtung nach Anspruch 1, wobei der Prozessor (10) konfiguriert ist, einen auf die Läsion zu richtenden Aktuator auf der Grundlage des berechneten Winkels der Biopsienadel (410) zu steuern.

3. Verarbeitungsvorrichtung nach Anspruch 1 oder 2, wobei der Prozessor (10) konfiguriert ist, eine Präsentationsverarbeitung durchzuführen, um einem Benutzer auf der Grundlage des berechneten Winkels der Biopsienadel (410) Informationen zur Unterstützung des Betriebs des Ultraschallendoskops (100) zu präsentieren.

4. Verarbeitungsvorrichtung nach einem der Ansprüche 1 bis 3, wobei der Prozessor (10) konfiguriert ist, einen Winkel und eine Tiefe der Biopsienadel (410) zum Einführen der Biopsienadel (410) in die Läsion auf der Grundlage des Bewegungsbereichs (R3) der Biopsienadel (410) und der Bereichsmarkierungsinformationen zu berechnen.

5. Verarbeitungsvorrichtung nach Anspruch 4, wobei der Prozessor (10) konfiguriert ist, einen Aktuator zu steuern, um die Biopsienadel (410) auf der Grundlage des berechneten Winkels und der Tiefe der Biopsienadel (410) elektrisch in die Läsion einzuführen.

6. Verarbeitungsvorrichtung nach Anspruch 4 oder 5, wobei der Prozessor (10) konfiguriert ist, eine Präsentationsverarbeitung durchzuführen, um einem Benutzer auf der Grundlage des berechneten Winkels und der Tiefe der Biopsienadel (410) Informationen zur Unterstützung des Betriebs des Ultraschallendoskops (100) zu präsentieren.

7. Verarbeitungsvorrichtung nach einem der Ansprüche 1 bis 6, wobei die Bereichsmarkierungsinformation eine Markierungsinformation, die einem Bereich der Läsion entspricht, und eine Markierungsinformationen, die einem Bereich eines vorbestimmten Gewebes entspricht, umfasst.

8. Verarbeitungsvorrichtung nach einem der Ansprüche 1 bis 7, die ferner einen Speicher (12) umfasst, der ein trainiertes Modell (22) speichert, das so trainiert ist, dass es zu dem von dem Ultraschallendoskop (100) aufgenommenen Ultraschallbild die Bereichsmarkierungsinformationen eines Erfassungsziels in dem Ultraschallbild ausgibt,
wobei der Prozessor (10) konfiguriert ist, die Bereichsmarkierungsinformationen auf der Grundlage des Ultraschallbilds und des trainierten Modells (22) zu erfassen.

9. Computerprogramm, das kofiguriert ist, von einer Verarbeitungsvorrichtung ausgeführt zu werden, wobei das Verfahren umfasst:
Erfassen eines Bildes, in dem eine Bereichsmarkierungsinformation einer Läsion in Bezug auf ein Ultraschallbild eines Ultraschallendoskops (100) mit einer Biopsienadel (410) gesetzt ist;
**gekennzeichnet durch**
Berechnen eines Winkels der Biopsienadel (410) zum Einführen der Biopsienadel (410) in die Läsion auf der Grundlage eines Bewegungsbereichs (R3) der Biopsienadel (410) und der Bereichsmarkierungsinformation;
**dadurch gekennzeichnet, dass** das Computerprogramm ferner umfasst:
Bestimmen, ob die Läsion in dem Bewegungsbereich (R3) enthalten ist; und
wenn die Läsion nicht in dem Bewegungsbereich (R3) enthalten ist, eine Befehlsinformation zum Ändern eines Winkels einer Sonde (150) des Ultraschallendoskops (100) auszugeben; und/oder
Bestimmen, ob die Läsion und ein vorbestimmtes Gewebe in dem Bewegungsbereich (R3) enthalten sind; und
wenn die Läsion in dem Bewegungsbereich (R3) enthalten ist und das vorbestimmte Gewebe nicht in dem Bewegungsbereich (R3) enthalten ist, Durchführen einer Steuerung zum Einführen der Biopsienadel (410) in die Läsion; und/oder
Bestimmen, ob ein vorbestimmtes Gewebe zwischen einer Projektionsposition der Biopsienadel (410) und der Läsion enthalten ist; und
wenn das vorbestimmte Gewebe zwischen der Projektionsposition der Biopsienadel (410) und der Läsion enthalten ist, Ausgeben einer Befehlsinformation zum Ändern eine Position einer Sonde (150) des Ultraschallendoskops (100).

10. Computerprogramm nach Anspruch 9, das umfasst:
Präsentieren von Informationen zur Unterstützung des Betriebs des Ultraschallendoskops (100) für einen Benutzer auf der Grundlage des berechneten Winkels der Biopsienadel (410); und/oder
Berechnen eines Winkels und einer Tiefe der Biopsienadel (410) zum Einführen der Biopsienadel (410) in die Läsion auf der Grundlage des Bewegungsbereichs (R3) der Biopsienadel (410) und der Bereichsmarkierungsinformation; und/oder
Anzeigen von Informationen zur Unterstützung des Betriebs des Ultraschallendoskops (100) für einen Benutzer auf der Grundlage des berechneten Winkels und der Tiefe der Biopsienadel (410).

11. Computerprogramm nach einem der Ansprüche 9 bis 10, wobei die Bereichsmarkierungsinformation eine Markierungsinformation, die einem Bereich der Läsion entspricht, und eine Markierungsinformationen, die einem Bereich eines vorbestimmten Gewebes entspricht, umfasst

12. Computerprogramm nach einem der Ansprüche 9 bis 11, das ferner umfasst
Erfassen der Bereichsmarkierungsinformation auf der Grundlage eines trainierten Modells (22), das so trainiert ist, dass zu dem von dem Ultraschallendoskop (100) aufgenommenen Ultraschallbild die Bereichsmarkierungsinformation eines Erfassungsziels in dem Ultraschallbild und das Ultraschallbild ausgibt.

## Revendications

1. Appareil de traitement comprenant:
un processeur (10) notamment un matériel, le processeur (10) étant configuré pour:
acquérir une image dans laquelle les informations de marquage de région d'une lésion sont définies par rapport à une image ultrasonique d'un endoscope à ultrasons (100) doté d'une aiguille à biopsie (410);
le processeur (10) étant configuré pour:
calculer un angle de l'aiguille à biopsie (410) pour insérer l'aiguille à biopsie (410) dans la lésion sur la base d'une plage mobile (R3) de l'aiguille à biopsie (410) et des informations de marquage de région;
**caractérisé en ce que** le processeur est en outre configuré pour:
déterminer si la lésion est incluse dans la plage mobile (R3); et
lorsque la lésion n'est pas incluse dans la plage mobile (R3), produire des informations d'instruction pour modifier l'angle d'une sonde (150) de l'endoscope à ultrasons (100); et/ou
dans lequel le processeur (10) est en outre configuré pour:
déterminer si la lésion et un tissu prédéterminé sont inclus dans la plage mobile (R3); et
lorsque la lésion est incluse dans la plage mobile (R3) et que le tissu prédéterminé n'est pas inclus dans la plage mobile (R3), procéder à l'insertion de l'aiguille à biopsie (410) dans la lésion; et/ou
dans lequel le processeur (10) est en outre configuré pour:
déterminer si un tissu prédéterminé se trouve entre une position de projection de l'aiguille à biopsie (410) et la lésion; et
lorsque le tissu prédéterminé se trouve entre la position de projection de l'aiguille à biopsie (410) et la lésion, produire des informations d'instruction pour modifier la position de la sonde (150) de l'endoscope à ultrasons (100).

2. Appareil de traitement selon la revendication 1, dans lequel le processeur (10) est configuré pour amener un actionneur à se diriger en direction de la lésion en fonction de l'angle calculé de l'aiguille à biopsie (410).

3. Appareil de traitement selon la revendication 1 ou 2, dans lequel le processeur (10) est configuré pour effectuer un processus de présentation pour présenter à un utilisateur les informations d'aide au fonctionnement de l'endoscope à ultrasons (100) sur la base de l'angle calculé de l'aiguille à biopsie (410).

4. Appareil de traitement selon l'une quelconque des revendications 1 à 3, dans lequel le processeur (10) est configuré pour calculer un angle et une profondeur de l'aiguille à biopsie (410) pour insérer l'aiguille à biopsie (410) dans la lésion sur la base de la plage mobile (R3) de l'aiguille à biopsie (410) et des informations de marquage de région.

5. Appareil de traitement selon la revendication 4, dans lequel le processeur (10) est configuré pour amener un actionneur à insérer électriquement l'aiguille à biopsie (410) dans la lésion en fonction de l'angle et de la profondeur calculés de l'aiguille à biopsie (410).

6. Appareil de traitement selon la revendication 4 ou 5, dans lequel le processeur (10) est configuré pour effectuer un processus de présentation pour présenter à un utilisateur des informations d'aide au fonctionnement de l'endoscope à ultrasons (100) sur la base de l'angle et de la profondeur calculés de l'aiguille à biopsie (410).

7. Appareil de traitement selon l'une quelconque des revendications 1 à 6, dans lequel les informations de marquage de région comprennent des informations de marquage correspondant à une région de la lésion et des informations de marquage correspondant à une région d'un tissu prédéterminé.

8. Appareil de traitement selon l'une quelconque des revendications 1 à 7, comprenant en outre une mémoire (12) qui stocke un modèle entraîné (22) entraîné de manière à produire, sur l'image ultrasonique capturée par l'endoscope à ultrasons (100), les informations de marquage de région d'une cible de détection dans l'image ultrasonique,
dans lequel le processeur (10) est configuré pour détecter les informations de marquage de région sur la base de l'image ultrasonique et du modèle entraîné (22).

9. Programme informatique configuré pour être exécuté par un appareil de traitement, le procédé comprenant:
l'acquisition d'une image dans laquelle les informations de marquage de région d'une lésion sont définies par rapport à une image ultrasonique d'un endoscope à ultrasons (100) doté d'une aiguille à biopsie (410);
le calcul d'un angle de l'aiguille à biopsie (410) pour insérer l'aiguille à biopsie (410) dans la lésion sur la base d'une plage mobile (R3) de l'aiguille à biopsie (410) et des informations de marquage de région;
**caractérisé en ce que** le programme informatique consiste en outre à:
déterminer si la lésion est incluse dans la plage mobile (R3); et lorsque la lésion n'est pas incluse dans la plage mobile (R3), produire des informations d'instruction pour modifier l'angle d'une sonde (150) de l'endoscope à ultrasons (100); et/ou
déterminer si la lésion et un tissu prédéterminé sont inclus dans la plage mobile (R3); et lorsque la lésion est incluse dans la plage mobile (R3) et que le tissu prédéterminé n'est pas inclus dans la plage mobile (R3), procéder à la commande d'insertion de l'aiguille à biopsie (410) dans la lésion; et/ou
déterminer si un tissu prédéterminé se trouve entre une position de projection de l'aiguille à biopsie (410) et la lésion; et lorsque le tissu prédéterminé se trouve entre la position de projection de l'aiguille à biopsie (410) et la lésion, produire des informations d'instruction pour modifier la position de la sonde (150) de l'endoscope à ultrasons (100).

10. Programme informatique selon la revendication 9, comprenant:
la présentation d'informations d'aide au fonctionnement de l'endoscope à ultrasons (100) à un utilisateur sur la base de l'angle calculé de l'aiguille à biopsie (410); et/ou
le calcul d'un angle et de la profondeur de l'aiguille à biopsie (410) pour insérer l'aiguille à biopsie (410) dans la lésion sur la base de la plage mobile (R3) de l'aiguille à biopsie (410) et des informations de marquage de région; et/ou
la présentation d'informations d'aide au fonctionnement de l'endoscope à ultrasons (100) à un utilisateur sur la base de l'angle et de la profondeur calculés de l'aiguille à biopsie (410).

11. Programme informatique selon l'une quelconque des revendications 9 à 10, dans lequel les informations de marquage de région comprennent des informations de marquage correspondant à une région de la lésion et des informations de marquage correspondant à une région d'un tissu important.

12. Programme informatique selon l'une quelconque des revendications 9 à 11, comprenant la détection des informations de marquage de région sur la base d'un modèle entraîné (22) entraîné de manière à produire, sur l'image ultrasonique capturée par l'endoscope à ultrasons (100), les informations de marquage de région d'une cible de détection dans l'image ultrasonique, et l'image ultrasonique.
